# DEMANDE DE BREVET EUROPEEN

(11) **EP 2 193 782 A1**
(43) Date de publication de la demande: **09.06.2010**
(21) Numéro de dépôt: 09174668.5
(22) Date de dépôt: 30.10.2009
(51) Int. Cl.: A61K 8/49, A61Q 17/04, A61K 8/35

(54) **Composition cosmetique contenant un derive de dibenzoylmethane et un compose ester de 2-pyrrolidinone 4-carboxy ; procede de photostabilisation du derive de dibenzoylmethane**

(30) Priorité: 08.12.2008 FR 0858335
(71) Demandeur: L'Oréal, 75008 Paris (FR)
(72) Inventeur: Richard, Hervé, 93320, Gagny (FR); Marat, Xavier, 75010, Paris (FR)
(74) Mandataire: Miszputen, Laurent

(57) **Abrégé**

La présente invention est relative à une composition comprenant dans un support cosmétiquement acceptable au moins un système filtrant UV, caractérisée par le fait qu'elle comprend :
(a) au moins un dérivé du dibenzoylméthane et
(b) au moins un composé ester de 2-pyrrolidinone 4-carboxy de formule (I) suivante :

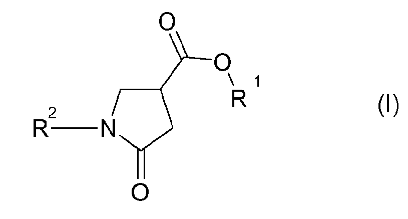

dans laquelle :
R¹ désigne un radical alkyle en C₁-C₂₀, linéaire ou ramifié.
R² désigne un radical alkyle en C₁-C₂₀, linéaire ou ramifié pouvant contenir un cycle en C₅-C₆, le radical phényle, le radical benzyle ou le radical phénéthyle.

Elle concerne également un procédé de photostabilisation vis-à-vis du rayonnement d'au moins un dérivé du dibenzoylméthane par une quantité efficace d'au moins un composé dithiolane de formule(I).

## Description

La présente invention est relative à une composition cosmétique contenant l'association d'au moins un filtre du type dérivé du dibenzoylméthane et d'au moins un composé ester de 2-pyrrolidinone 4-carboxy de formule (I) dont on donnera la définition ci-après.

Elle concerne également un procédé de photostabilisation vis-à-vis du rayonnement d'au moins un filtre du type dérivé du dibenzoylméthane par un composé ester de 2-pyrrolidinone 4-carboxy particulier de formule (I) dont on donnera la définition ci-après.

La présente invention est relative également à l'utilisation d'au moins un composé ester de 2-pyrrolidinone 4-carboxy particulier de formule (I) dans une composition comprenant dans un support cosmétiquement acceptable, au moins un dérivé du dibenzoylméthane dans le but de d'améliorer l'efficacité de ladite composition vis-à-vis des rayons UV-A.

On sait que les radiations lumineuses de longueurs d'onde comprises entre 280 nm et 400 nm permettent le brunissement de l'épiderme humain, et que les rayons de longueurs d'onde plus particulièrement comprises entre 280 et 320 nm, connus sous la dénomination UV-B, provoquent des érythèmes et des brûlures cutanées qui peuvent nuire au développement du bronzage naturel. Pour ces raisons ainsi que pour des raisons esthétiques, il existe une demande constante de moyens de contrôle de ce bronzage naturel en vue de contrôler ainsi la couleur de la peau ; il convient donc de filtrer ce rayonnement UV-B.

On sait également que les rayons UV-A, de longueurs d'onde comprises entre 320 et 400 nm, qui provoquent le brunissement de la peau, sont susceptibles d'induire une altération de celle-ci, notamment dans le cas d'une peau sensible ou d'une peau continuellement exposée au rayonnement solaire. Les rayons UV-A provoquent en particulier une perte d'élasticité de la peau et l'apparition de rides conduisant à un vieillissement cutané prématuré. Ils favorisent le déclenchement de la réaction érythémateuse ou amplifient cette réaction chez certains sujets et peuvent même être à l'origine de réactions phototoxiques ou photo-allergiques. Ainsi, pour des raisons esthétiques et cosmétiques telles que la conservation de l'élasticité naturelle de la peau par exemple, de plus en plus de gens désirent contrôler l'effet des rayons UV-A sur leur peau. Il est donc souhaitable de filtrer aussi le rayonnement UV-A.

Dans le but d'assurer une protection de la peau et des matières kératiniques contre le rayonnement UV, on utilise généralement des compositions antisolaires comprenant des filtres organiques, actifs dans l'UV-A et actifs dans l'UV-B. La majorité de ces filtres est liposoluble.

A cet égard, une famille de filtres UV-A particulièrement intéressante est actuellement constituée par les dérivés du dibenzoylméthane, et notamment le 4-ter-butyl-4'-méthoxydibenzoyl méthane, qui présentent en effet un fort pouvoir d'absorption intrinsèque. Ces dérivés du dibenzoylméthane, qui sont maintenant des produits bien connus en soi à titre de filtres actifs dans les UV-A, sont notamment décrits dans les demandes de brevets français FR-A-2326405 et

FR-A-2440933, ainsi que dans la demande de brevet européen EP-A-0114607 ; le 4-ter-butyl- 4'-méthoxydibenzoylméthane est par ailleurs actuellement proposé à la vente sous la dénomination commerciale de « PARSOL 1789 ® » par la Société DSM NUTRITIONAL PRODUCTS.

Malheureusement, il se trouve que les dérivés du dibenzoylméthane sont des produits relativement sensibles au rayonnement ultraviolet (surtout UV-A), c'est-à-dire, plus précisément, qu'ils présentent une fâcheuse tendance à se dégrader plus ou moins rapidement sous l'action de ce dernier. Ainsi, ce manque substantiel de stabilité photochimique des dérivés du dibenzoylméthane face au rayonnement ultraviolet auquel ils sont par nature destinés à être soumis, ne permet pas de garantir une protection constante durant une exposition solaire prolongée, de sorte que des applications répétées à intervalles de temps réguliers et rapprochés doivent être effectuées par l'utilisateur pour obtenir une protection efficace de la peau contre les rayons UV.

On sait que dans le brevet EP717982 les composés amides ont un effet photostabilisant sur les dérivés de dibenzoylméthane et plus particulièrement les huiles amidées N,N-disubstituées comme le composé N,N-diéthyl-3-méthylbenzamide de structure : 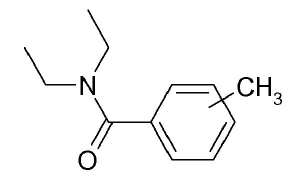 ou le N-butyl, N-acétyl aminopropionate d'éthyle de formule : 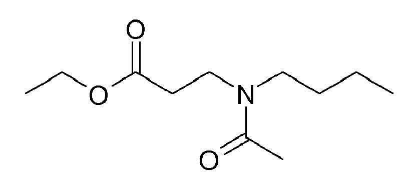 comme le produit vendu sous la dénomination commerciale R3535 par la société MERCK. Ces huiles amidées ont été décrites notamment dans la demande US2007141014 comme solvants dans des formulations cosmétiques d'actifs difficilement solubles dans les huiles comme les filtres UV, les dérivés de flavone, les dérivés de chromone, les aryloximes et les parabenes.

Cependant cette stabilisation est obtenue en présence de 20 à 30% de ce dernier composé lequel présente un pouvoir solvant important pour l'ensemble des matières premières mises en jeu pour réaliser les formulations ce qui a pour conséquence de se traduire par une déstabilisation des compositions, rendant impropres à l'utilisation les compositions contenant une telle association.

On connaît également dans le brevet US6,528,068 des compositions solaires comprenant des huiles amidées qui sont des esters d'acide N-acyl-aminé neutres comprenant un groupe acyle à longue chaîne linéaire ou ramifiée en C₆-C₂₂ comme l'Isopropyl Lauroyl Sarcosinate (ELDEW SL 205 d'AJINOMOTO) associées à des filtres organiques UV difficilement solubles dans les huiles habituellement utilisées dans les formulations solaires. L'association de ces huiles amidées avec un dérivé de dibenzoylméthane comme le 4-ter-butyl- 4'-méthoxydibenzoyl méthane ne permet pas d'obtenir une photostabilité de dibenzoylméthane pleinement satisfaisante.

La photostabilisation des dérivés du dibenzoylméthane vis-à-vis du rayonnement UV par des composés amidés constitue donc, à ce jour, un problème qui n'a pas encore été résolu de manière complètement satisfaisante.

Or, la Demanderesse vient maintenant de découvrir, de façon surprenante, qu'en associant aux dérivés du dibenzoylméthane mentionnés ci-dessus un composé ester de 2-pyrrolidinone 4-carboxy particulier de formule (I) que l'on définira plus loin en détail, il était possible d'améliorer encore de manière substantielle et remarquable, la stabilité photochimique (ou photostabilité) de ces mêmes dérivés du dibenzoylméthane et leur efficacité dans l'UV-A. Les compositions contenant une telle association conduisent également après application à une répartition plus homogène du filtre dibenzoylméthane.

Cette découverte est à la base de la présente invention.

Ainsi, conformément à l'un des objets de la présente invention, il est maintenant proposé une composition comprenant dans un support cosmétiquement acceptable au moins un système filtrant UV, caractérisée par le fait qu'elle comprend :
(a) au moins un dérivé du dibenzoylméthane et
(b) au moins un composés ester de 2-pyrrolidinone 4-carboxy de formule (I) dont on donnera la définition ci-après.

Un autre objet de l'invention concerne également un procédé pour améliorer la stabilité chimique vis-à-vis du rayonnement UV d'au moins un dérivé du dibenzoylméthane consistant à associer audit dérivé de dibenzoylméthane une quantité efficace d'au moins un composé ester de 2-pyrrolidinone 4-carboxy de formule (I) dont on donnera les définitions ci-après

La présente invention a également pour objet l'utilisation d'au moins un composé ester de 2-pyrrolidinone 4-carboxy de formule (I) dans une composition comprenant dans un support cosmétiquement acceptable, au moins un dérivé du dibenzoylméthane dans le but d'améliorer l'efficacité de ladite composition vis-à-vis des rayons UV-A.

D'autres caractéristiques, aspects et avantages de l'invention apparaîtront à la lecture de la description détaillée qui va suivre.

Par "cosmétiquement acceptable", on entend compatible avec la peau et/ou ses phanères, qui présente une couleur, une odeur et un toucher agréables et qui ne génère pas d'inconforts inacceptables (picotements, tiraillements, rougeurs), susceptibles de détourner la consommatrice d'utiliser cette composition.

Par « quantité efficace », on entend une quantité suffisante pour obtenir une amélioration notable et significative de la photostabilité du ou des dérivés du dibenzoylméthane dans la composition cosmétique. Cette quantité minimale en composé ester de 2-pyrrolidinone 4-carboxy de formule (I), qui peut varier selon la nature du support retenu pour la composition, peut être déterminée sans aucune difficulté au moyen d'un test classique de mesure de photostabilité, tel que celui donné dans les exemples ci-après.

Les composés esters de 2-pyrrolidinone 4-carboxy conformes à l'invention sont choisis par ceux répondant à la formule générale (I) suivante : 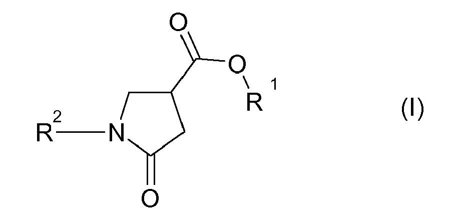 dans laquelle :
R¹ désigne un radical alkyle en C₁-C₂₀, linéaire ou ramifié.
R² désigne un radical alkyle en C₁-C₂₀, linéaire ou ramifié pouvant contenir un cycle en C₅-C₆, le radical phényle, le radical benzyle ou le radical phénéthyle.

Dans la formule (I), parmi les groupes alkyles, on peut notamment citer les groupes méthyle, éthyle, n-propyle, isopropyle, n-butyle, iso-butyle, tert-butyle, n-octyle, éthyle-2 hexyle, dodécyle, héxadécyle, cyclohexyle ou méthyle cyclohexyle.

Parmi les composés de formule (I), on utilisera plus particulièrement les produits (a) à (oo) suivants :

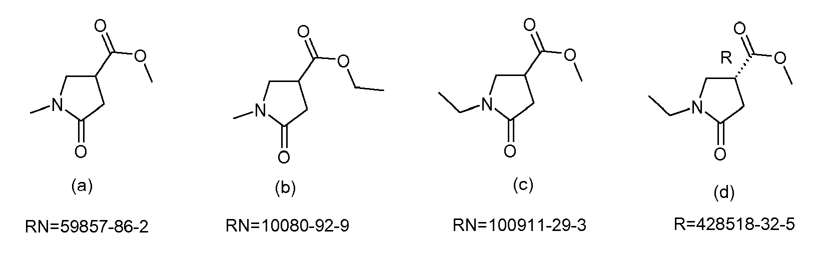

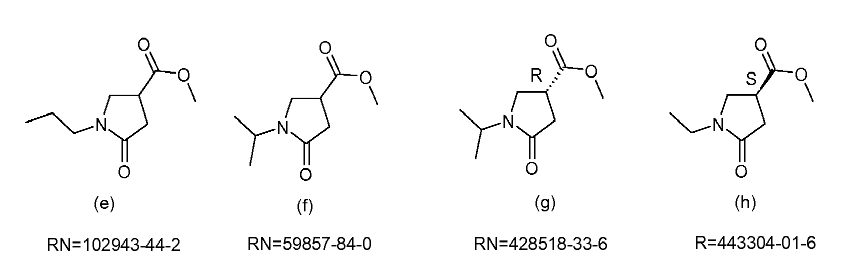

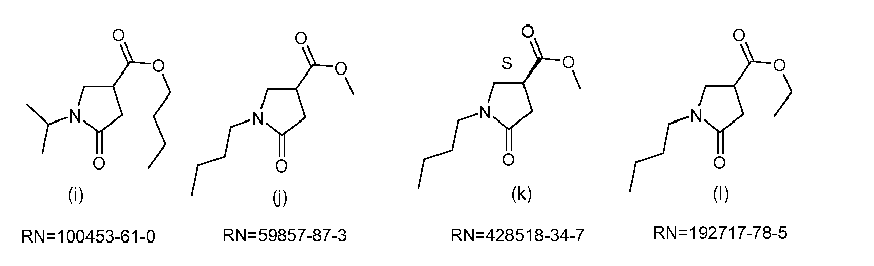

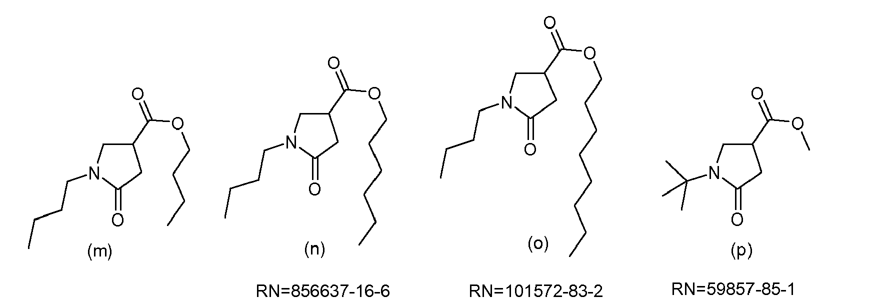

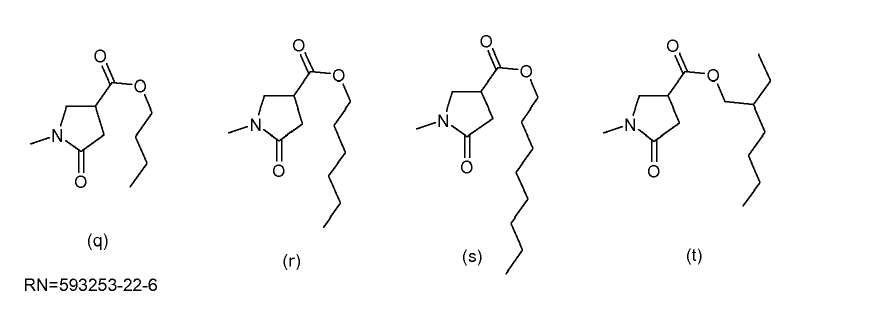

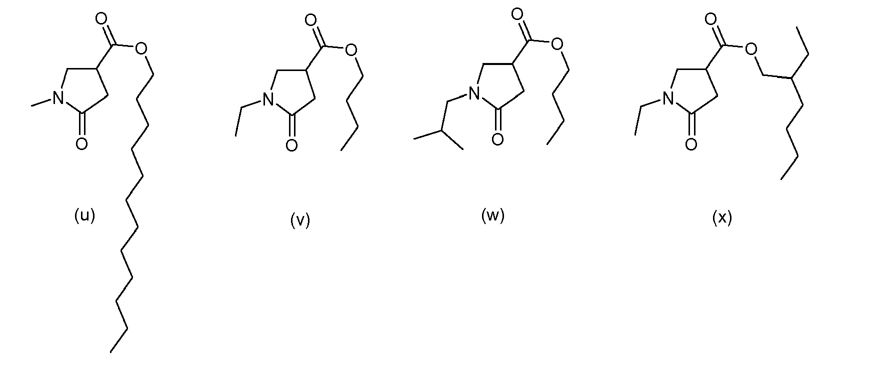

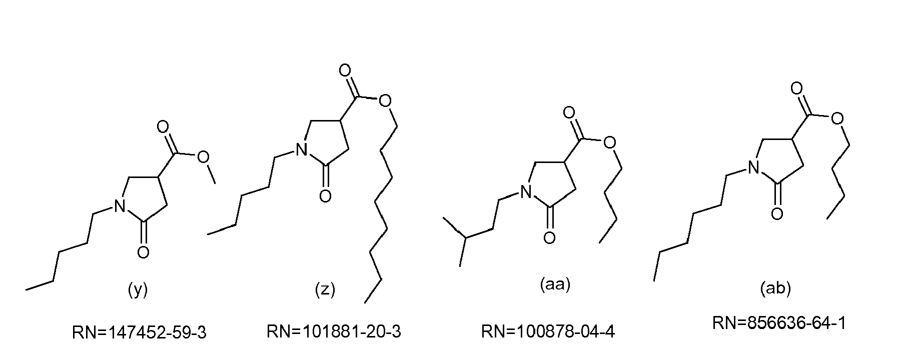

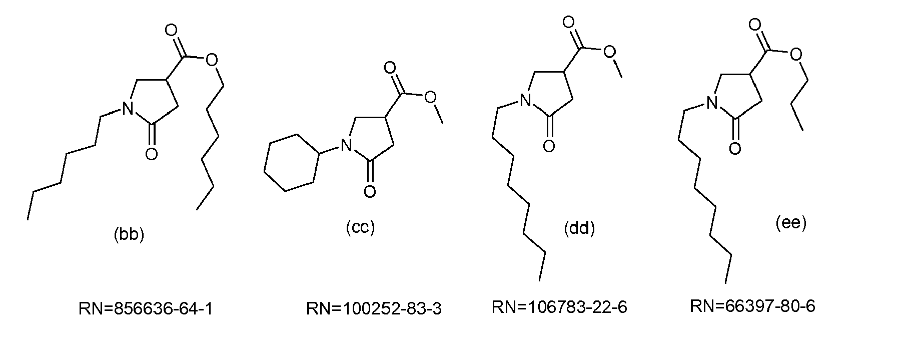

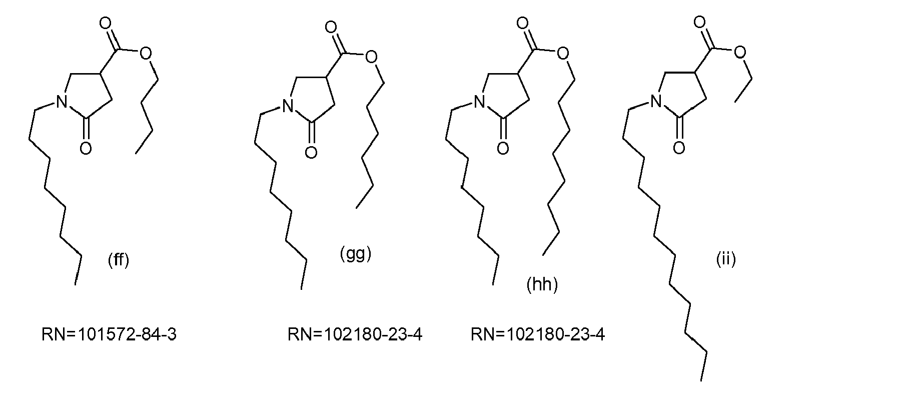

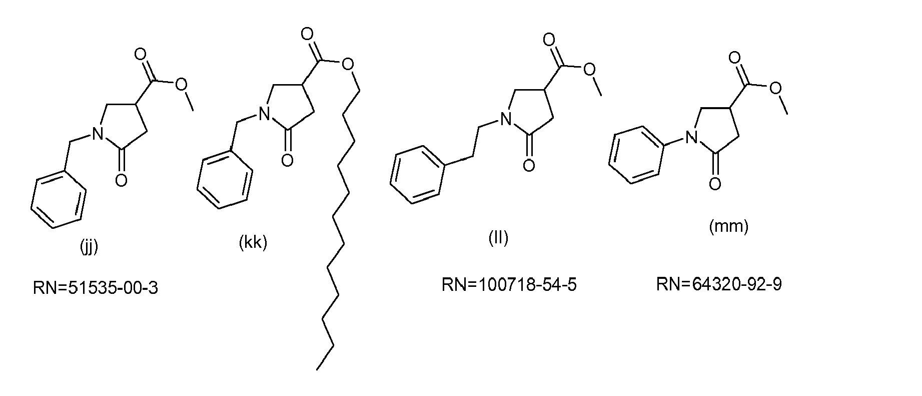

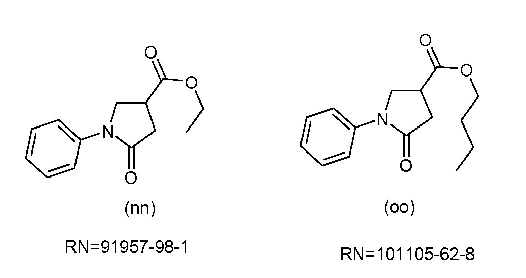

On préférera plus particulièrement les composés (j) (l) (m) et (n).

Les dérivés de formule (I), dont les synthèses sont décrites dans les articles suivants : J. Org. Chem., 26, pages 1519-24 (1961) ; Tetrahedron Asymmetric, 12 (23), pages 3241-9 (2001) ; J. Industrial & Engineering Chem., 47, pages 1572-8 (1955) ; J. Am. Chem. Soc., 60, pages 402-6 (1938) ; et dans les brevets EP 0069512, US 2811496 (1955), US 2826588, US 3136620, FR 2290199 et FR 2696744 peuvent être facilement obtenus :
- soit par la condensation d'un diester de l'acide itaconique de formule (II) avec une amine primaire de formule (III), avec ou sans solvant à une température comprise entre 20°C et 150°C selon le schéma suivant : 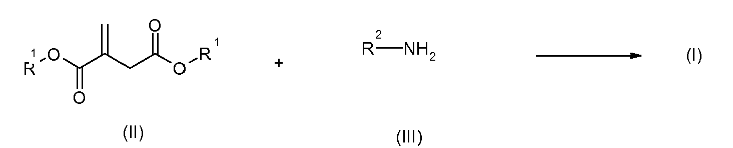
- soit en 2 étapes à partir de l'acide itaconique de formule (IV) par condensation avec l'amine primaire de formule (III) en présence d'un solvant ou pas pour donner l'acide intermédiaire de formule (V), suivi par une estérification de cet acide de formule (V) en présence d'un excès d'alcool de formule (VI) selon le schéma suivant :

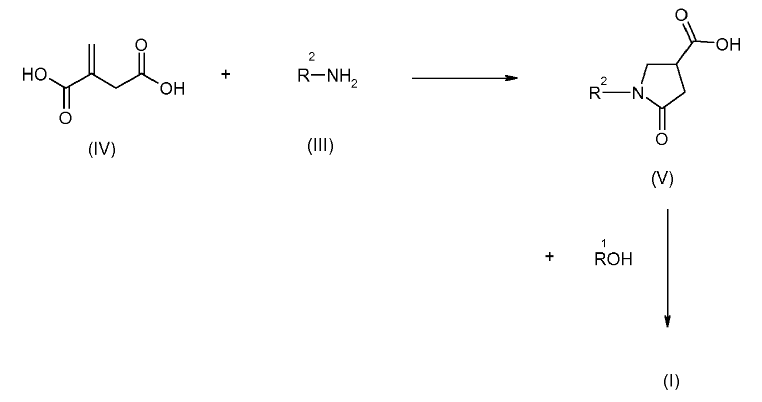

Les composés esters de 2-pyrrolidinone 4-carboxy de formule (I) conformes à l'invention sont de préférence présents dans les compositions à des teneurs de 0,01 à 20% en poids et plus préférentiellement de 0,1 à 10 % en poids par rapport au poids total de la composition.

Parmi les dérivés du dibenzoylméthane, on peut notamment citer, de manière non limitative :
- le 2-méthyldibenzoylméthane,
- le 4-méthyldibenzoylméthane,
- le 4-isopropyldibenzoylméthane,
- le 4-tert.-butyldibenzoylméthane,
- le 2,4-diméthyldibenzoylméthane,
- le 2,5-diméthyldibenzoylméthane,
- le 4,4'-diisopropyldibenzoylméthane,
- le 4,4'-diméthoxydibenzoylméthane,
- le 4-tert.-butyl-4'-méthoxydibenzoylméthane,
- le 2-méthyl-5-isopropyl-4'-méthoxydibenzoylméthane,
- le 2-méthyl-5-tert-butyl-4'-méthxydibenzoylméthane,
- le 2,4-diméthyl-4'-méthoxydibenzoylméthane,
- le 2,6-diméthyl-4-tert-butyl-4'-méthoxydibenzoylméthane.

Parmi les dérivés du dibenzoylméthane mentionnés ci-dessus, on utilisera en particulier le 4-isopropyl-dibenzoylméthane, vendu sous la dénomination de "EUSOLEX 8020" par la Société MERCK, et répondant à la formule suivante :

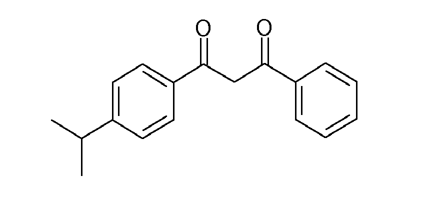

On peut citer également le 1-(4-methoxy-1-benzofuran-5-yl)-3-phenylpropane-1,3-dione, proposé à la vente par la société QUEST sous le nom de PONGAMOL de formule :

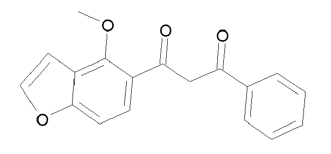

On préfère tout particulièrement mettre en oeuvre le 4-(ter.-butyl) 4'-méthoxy dibenzoylméthane ou Butyl Methoxy Dibenzoylmethane, proposé à la vente sous la dénomination commerciale de "PARSOL 1789" par la Société DSM NUTRITIONAL PRODUCTS ; ce filtre répond à la formule suivante :

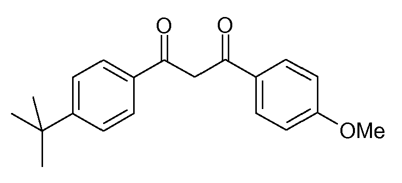

Le ou les dérivés du dibenzoylméthane peuvent être présents dans les compositions conformes à l'invention à des teneurs qui varient de préférence de 0,01 à 10% en poids et plus préférentiellement de 0,1 à 6% en poids par rapport au poids total de la composition.

Selon une forme particulièrement préférée de l'invention, on utilisera dans les compositions de l'invention en plus un composé s-triazine silicié et substitué par deux groupements aminobenzoates ou aminobenzamides de formule générale (VI) suivante ou l'une de ses formes tautomères : 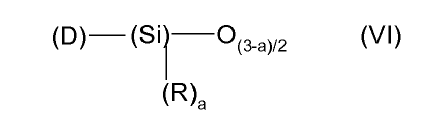 dans laquelle
- R, identiques ou différents représentent un radical alkyle en C₁-C₃₀, linéaire ou ramifié et éventuellement halogéné ou insaturé, un radical aryle en C₆-C₁₂, un radical alkoxy en C₁-C₁₀, un radical hydroxy ou le groupe triméthylsilyloxy ;
- a = 0 à 3 ; en plus des unités de formule -A-(Si)(R)ₐ(O)_{(3-a)/2}, l'organosiloxane peut comporter des unités de formule : (R)_{b}-(Si)(O)_{(4-b)/2} dans lesquelles : R a la même signification que dans la formule (II), b = 1, 2 ou 3 ;
- le groupe (D) désigne un composé s-triazine de formule (VII) suivante : 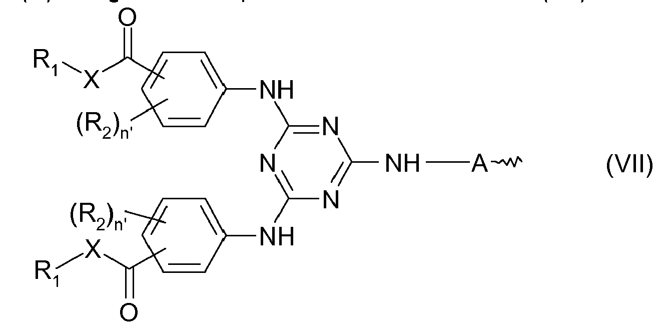 où
   - X représente -O- ou -NR₃-, avec R₃ qui représente l'hydrogène ou un radical alkyle en C₁-C₅,
   - R₁ représente un radical alkyle en C₁-C₃₀, linéaire ou ramifié et éventuellement insaturé et pouvant contenir un atome de silicium, un groupe cycloalkyle en C₅-C₂₀, éventuellement substitué par 1 à 3 radicaux alkyles en C₁-C₄, linéaires ou ramifiés, le groupe -(CH₂CHR₄-O)ₘR₅ ou le groupe -CH₂-CH(OH)-CH₂-O-R₆,
   - R₄ représente l'hydrogène ou méthyle ; le groupement (C=O)XR, pouvant être en position ortho, méta ou para du groupement amino,
   - R₅ représente l'hydrogène ou un groupe alkyle en C₁-C₈,
   - R₆ représentent l'hydrogène ou un groupe alkyle en C₄-C₈,
   - m est un nombre entier allant de 2 à 20,
   - n' = 0 à 2,
   - R₂, identiques ou différents, représentent un radical hydroxy, un radical alkyle en C₁-C₈, linéaire ou ramifié, un radical alcoxy en C₁-C₈, deux R₂ adjacents d'un même noyau aromatique pouvant former ensemble un groupement alkylidène dioxy dans lequel le groupe alkylidène contient 1 ou 2 atomes de carbone,
   - A est un radical divalent choisi parmi méthylène, -[CH(Si(CH₃)₃]-, éthylène ou un groupe répondant à l'une des formules (VIII), (IX), (X) ou (XI) suivantes : 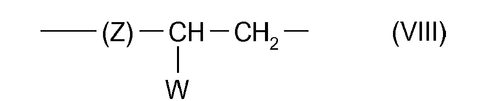 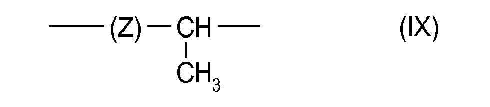 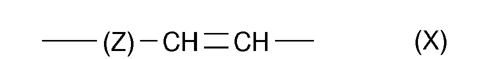 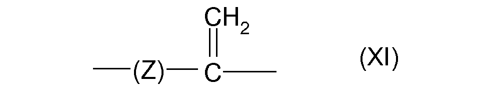
   dans lesquelles :
   - Z est un diradical alkylène en C₁-C₁₀, linéaire ou ramifié, saturé ou insaturé, éventuellement substitué par un radical hydroxyle ou des oxygènes et pouvant éventuellement contenir un groupement amino,
   - W représente un atome d'hydrogène, un radical hydroxyle ou un radical alkyle en C₁-C₈, linéaire ou ramifié, saturé ou insaturé.

Il est à noter que les dérivés de formule (VI) peuvent être utilisés sous leurs formes tautomères et plus particulièrement sous la forme tautomère de formule (VI') suivante : 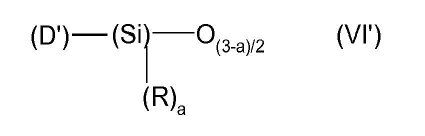 dans laquelle le groupe (D') désigne un composé s-triazine de formule (VII') suivante :

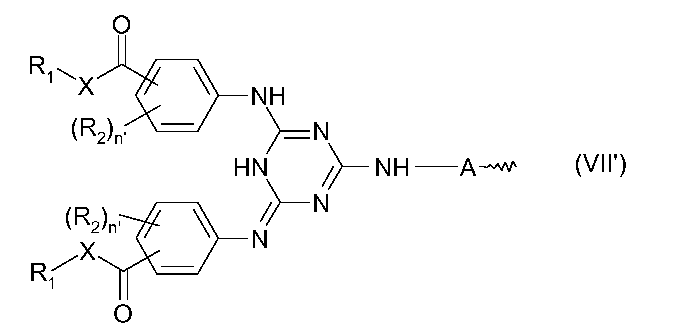

Les dérivés de s-triazine préférentiels sont ceux pour lesquels dans la formule (VII) ou (VII') au moins l'une, et encore plus préférentiellement l'ensemble, des caractéristiques suivantes sont remplies :
R est méthyle,
a = 1 ou 2,
X est O,
R₁ est un radical en C₄-C₅
n'=0,
le groupement (C=O)XR₁ est en position para vis-à-vis du groupement amino,
Z = -CH₂-,
W = H.

De manière préférée, les composés s-triazine de l'invention sont représentés par les formules (Vla), (Vlb) ou (Vlc) suivantes :

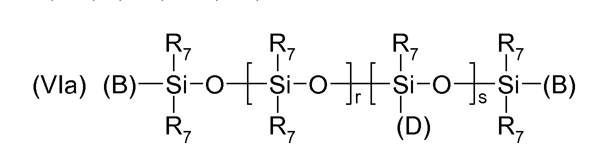

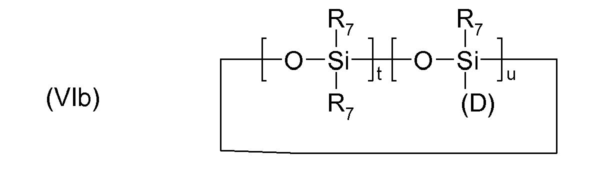

(D)-Si(R₈)₃ (Vlc)

dans lesquelles :
- (D) répond à la formule (VII) telle que définie ci-dessus,
- R₇, identiques ou différents, sont choisis parmi les radicaux alkyles linéaires ou ramifiés en C₁-C₂₀, phényle, 3,3,3-trifluoropropyle et triméthylsilyloxy ou le radical hydroxy,
- R₈, identiques ou différents, sont choisis parmi les radicaux alkyles et alcényles, linéaires ou ramifiés en C₁-C₂₀ , les radicaux hydroxy ou phényle,
- (B), identiques ou différents sont choisis parmi les radicaux R₇ et le radical (D), r est un nombre entier compris entre 0 et 200 inclusivement,
- s est un nombre entier allant de 0 à 50 et si s = 0, au moins l'un des deux symboles (B) désigne (D),
- u est un nombre entier allant de 1 à 10,
- t est un nombre entier allant de 0 à 10, étant entendu que t + u est égal ou supérieur à 3 ainsi que leurs formes tautomères.

Les diorganosiloxanes linéaires de formule (Vla) sont particulièrement préférés.

Les diorganosiloxanes linéaires ou cycliques de formule (Vla) ou (Vlb) rentrant dans le cadre de la présente invention, sont des oligomères ou polymères statistiques présentant de préférence au moins l'une, et encore plus préférentiellement l'ensemble, des caractéristiques suivantes :
- R₇ est le radical méthyle ou le radical hydroxy
- B est préférentiellement méthyle (cas des composés linéaires de formule (Va)),

A titre d'exemples de composés de formule (VI) particulièrement préférés, on citera les composés de formules (1) à (26) suivantes ainsi que leurs formes tautomères :

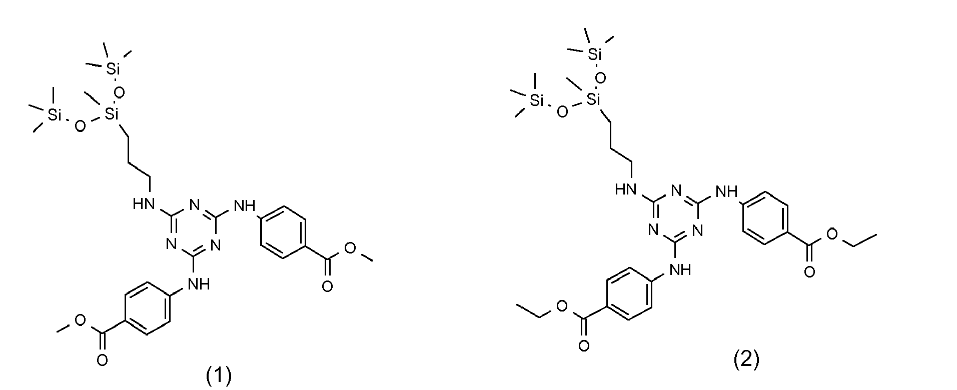

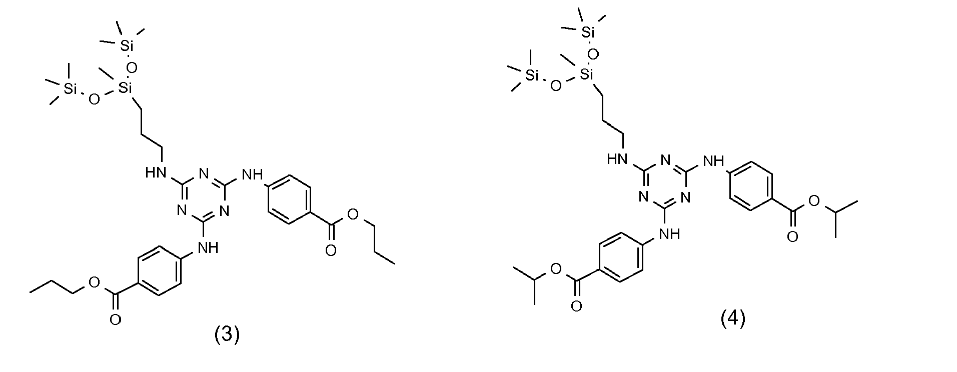

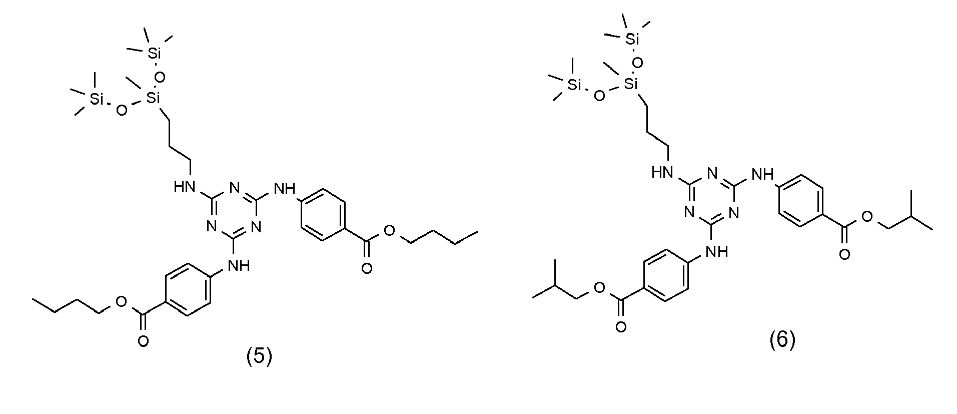

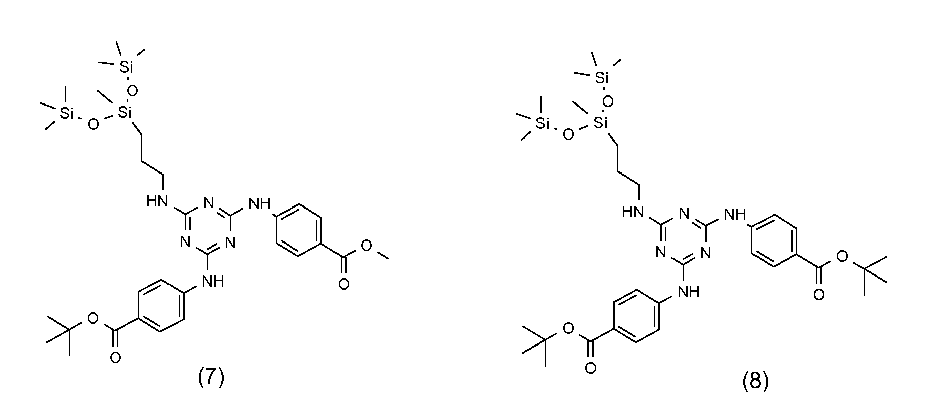

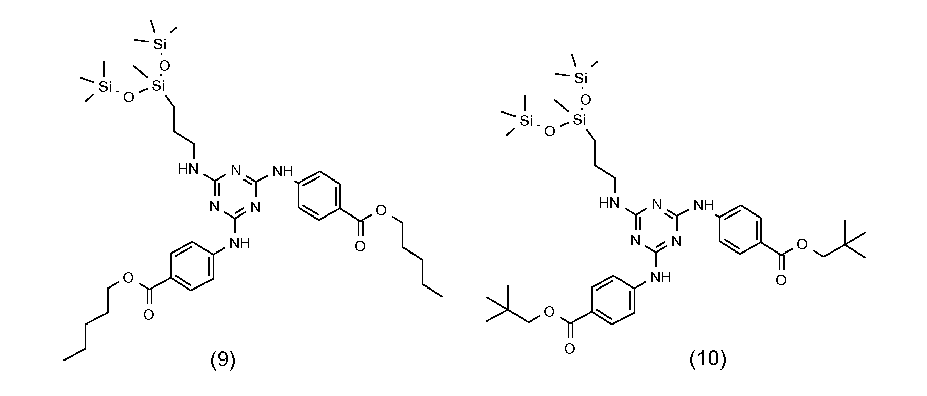

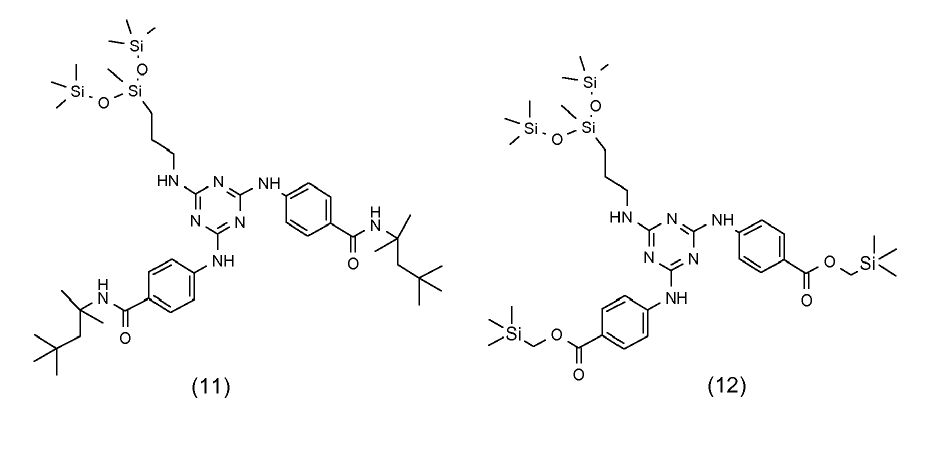

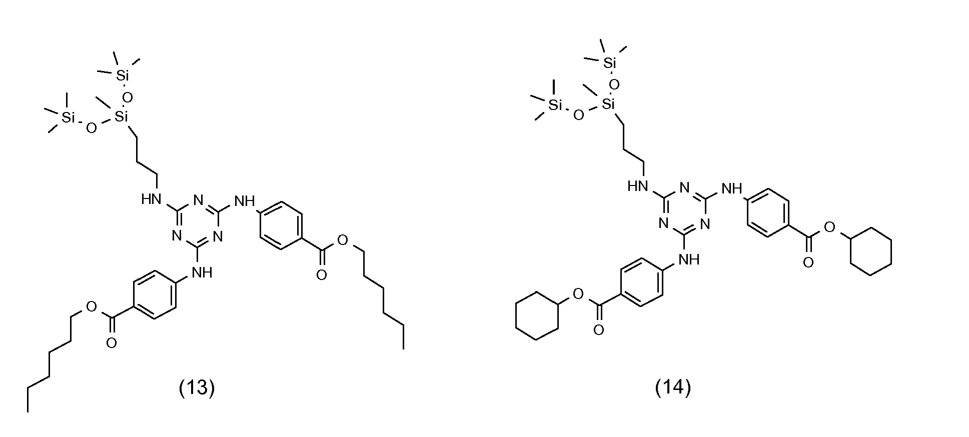

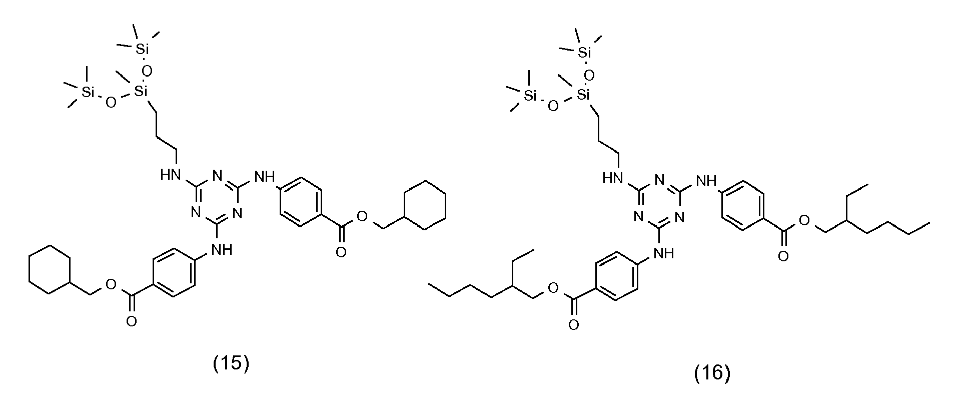

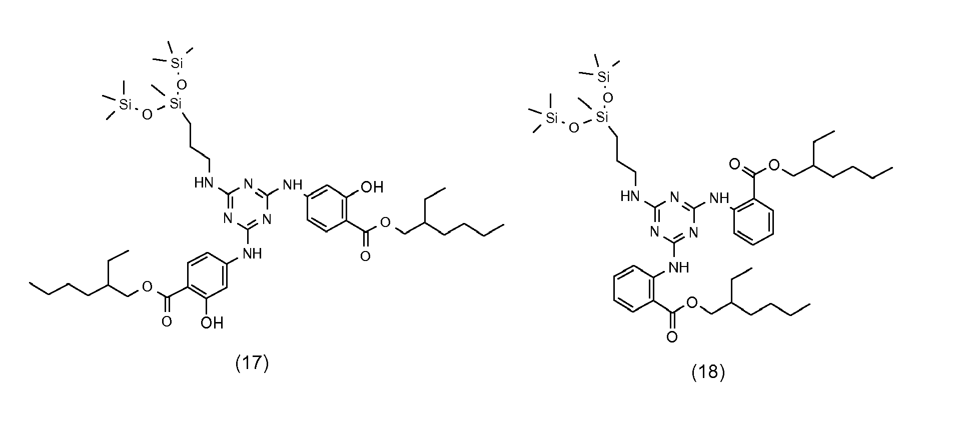

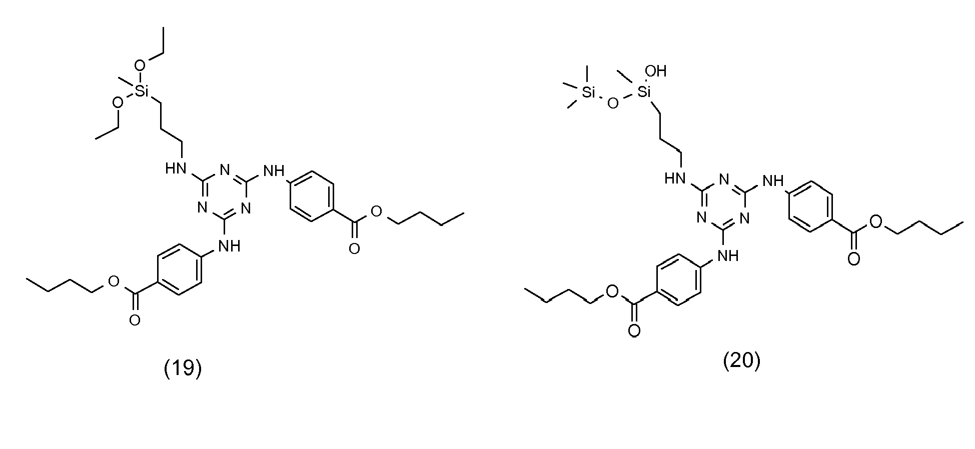

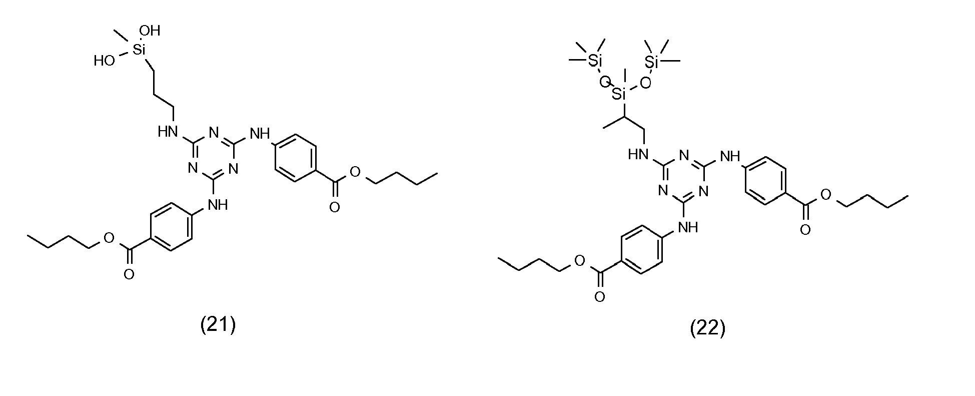

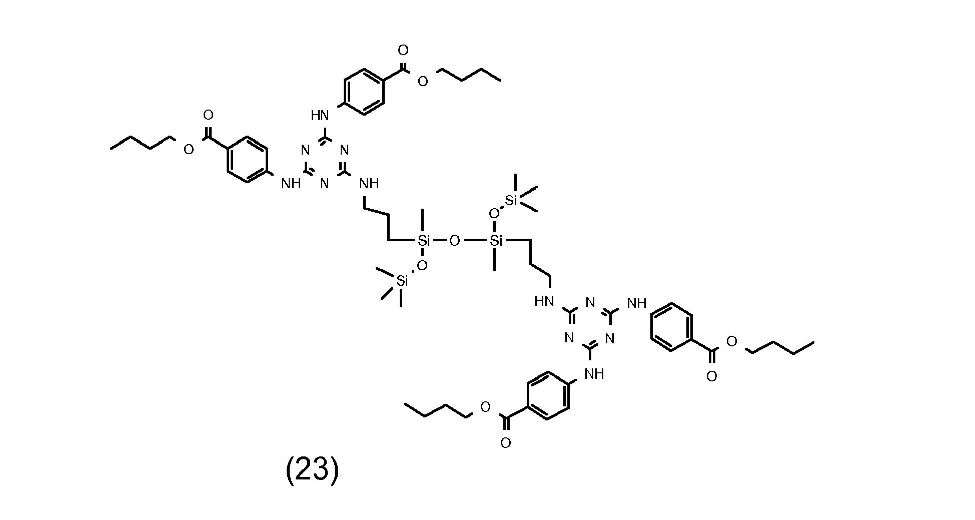

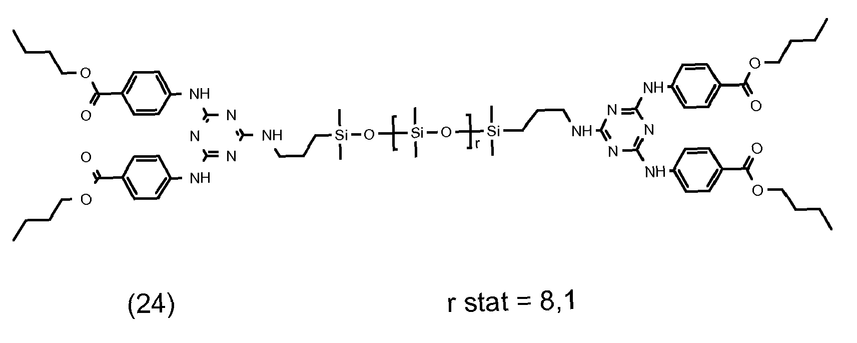

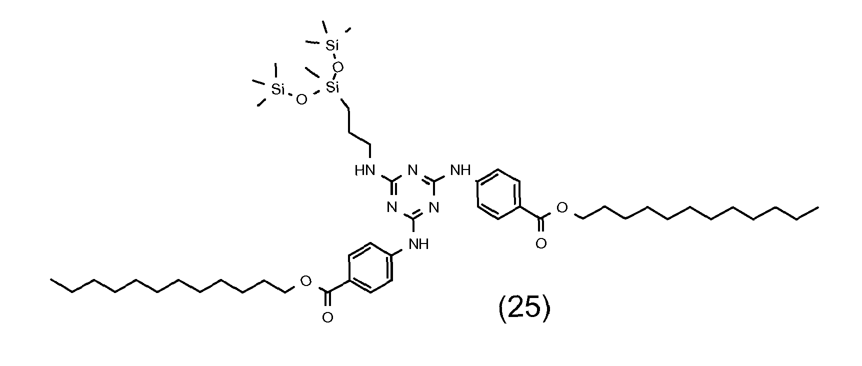

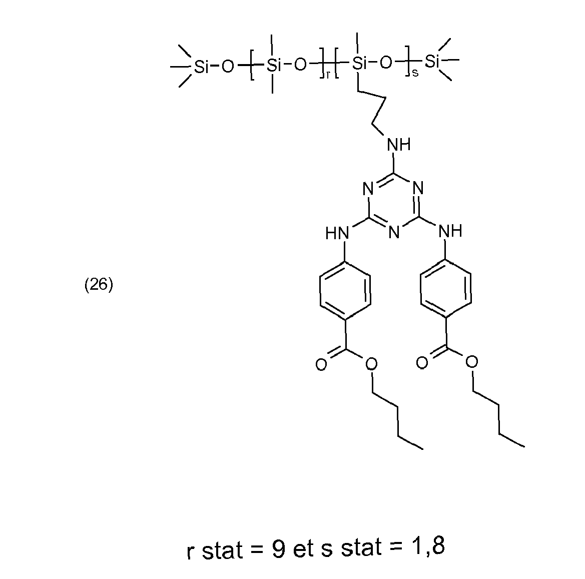

On utilisera plus particulièrement les composés choisis parmi
- la 2,4-bis(4'-aminobenzoate d'éthyle)-6-[(3-{1,3,3,3-tetramethyl-1-[(trimethylsilyl)oxy]disiloxanyl}propyl)amino]-s-triazine de formule (2), 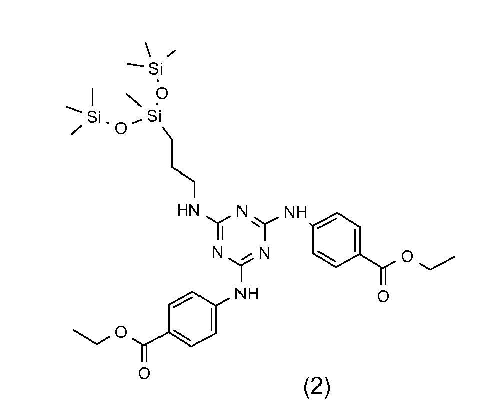
- la 2,4-bis(4'-aminobenzoate d'isopropyle)-6-[(3-{1,3,3,3-tetramethyl-1-[(trimethylsilyl)oxy]disiloxanyl}propyl)amino]-s-triazine de formule (4) 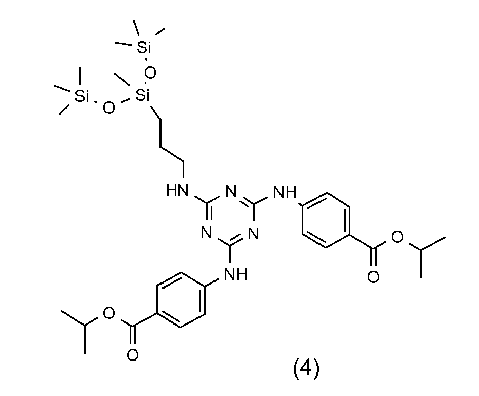
- la 2,4-bis(4'-aminobenzoate de n-butyle)-6-[(3-{1,3,3,3-tetramethyl-1-[(trimethylsilyl)oxy]disiloxanyl}propyl)amino]-s-triazine de formule (5) 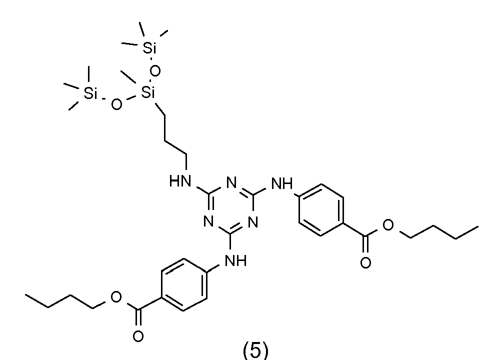
- la 2,4-bis(4'-aminobenzoate d'isobutyle)-6-[(3-{1,3,3,3-tetramethyl-1-[(trimethylsilyl)oxy]disiloxanyl}propyl)amino]-s-triazine de formule (6) 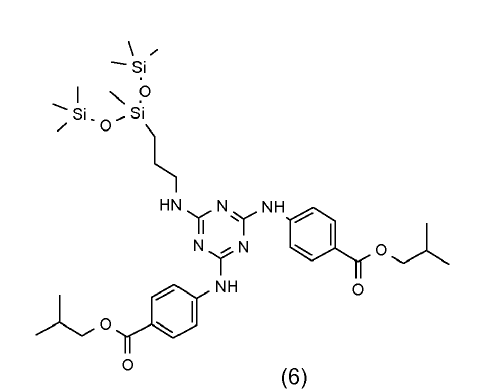
- la 2,4-bis(4'-aminobenzoate de néopentyle)- 6-[(3-{1,3,3,3-tetramethyl-1-[(trimethylsilyl)oxy]disiloxanyl}propyl)amino]-s-triazine de formule (10).

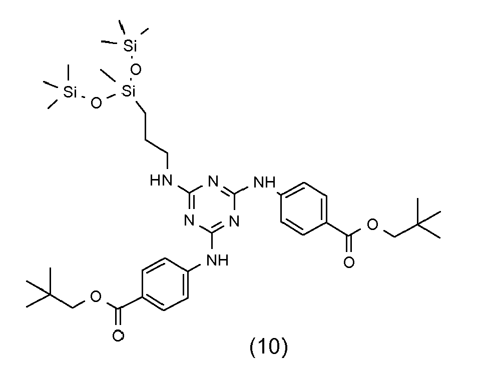

On utilisera encore plus particulièrement la 2,4-bis(4'-aminobenzoate de n-butyle)-6-[(3-{1,3,3,3-tetramethyl-1-[(trimethylsilyl)oxy]disiloxanyl}propyl)amino]-s-triazine de formule (5) :

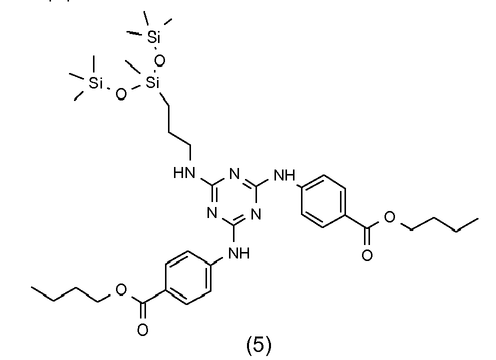

Parmi les composés triazines de formule (II) et leurs formes tautomères, certains sont connus et ont été décrits dans les brevets EP 0841341 et FR 2886143.

Les compositions conformes à l'invention peuvent comporter en plus d'autres filtres UV organiques complémentaires actifs dans l'UVA et/ou l'UVB hydrosolubles ou liposolubles ou bien insolubles dans les solvants cosmétiques couramment utilisés.

Bien entendu, l'homme de l'art veillera à choisir le ou les éventuels filtres complémentaires et/ou leurs quantités de manière telle que les propriétés avantageuses attachées intrinsèquement aux compositions conformes à l'invention ne soient pas, ou substantiellement pas, altérées par la ou les adjonctions envisagées notamment l'amélioration de la photostabilité du dérivé de dibenzoylméthane.

Les filtres organiques complémentaires sont notamment choisis parmi les anthranilates ; les dérivés cinnamiques ; les dérivés salicyliques, les dérivés du camphre ; les dérivés de la benzophénone ; les dérivés de β , β - diphénylacrylate ; les dérivés de triazine ; les dérivés de benzotriazole ; les dérivés de benzalmalonate notamment ceux cités dans le brevet US5624663 ; les dérivés de benzimidazole ; les imidazolines ; les dérivés bis-benzoazolyle tels que décrits dans les brevets EP669323 et US 2,463,264; les dérivés de l'acide p-aminobenzoïque (PABA) ; les dérivés de méthylène bis-(hydroxyphényl benzotriazole) tels que décrits dans les demandes US5,237,071, US 5,166,355, GB2303549, DE 197 26 184 et EP893119 ; les dérivés de benzoxazole tels que décrits dans les demandes de brevet EP0832642, EP1027883, EP1300137 et DE10162844 ; les polymères filtres et silicones filtres tels que ceux décrits notamment dans la demande WO-93/04665 ; les dimères dérivés d'α-alkylstyrène tels que ceux décrits dans la demande de brevet DE19855649 ; les 4,4-diarylbutadiènes tels que décrits dans les demandes EP0967200, DE19746654, DE19755649, EP-A-1008586, EP1133980 et EP133981 ; les dérivés de mérocyanines tels que ceux décrits dans les demandes W004006878, W005058269 et W006032741 et leurs mélanges .

Comme exemples d'agents photoprotecteurs organiques complémentaires, on peut citer ceux désignés ci-dessous sous leur nom INCI :

### Dérivés cinnamiques :

Ethylhexyl Methoxycinnamate vendu notamment sous le nom commercial PARSOL MCX par DSM Nutritional Products, Inc"
Isopropyl Methoxycinnamate
Isoamyl Methoxycinnamate vendu sous le nom commercial NEO HELIOPAN E 1000 par SYMRISE,
DEA Methoxycinnamate,
Diisopropyl Methylcinnamate,
Glyceryl Ethylhexanoate Dimethoxycinnamate

### Dérivés de l'acide para-aminobenzoique :

PABA,
Ethyl PABA,
Ethyl Dihydroxypropyl PABA,
Ethylhexyl Diméthyl PABA vendu notamment sous le nom « ESCALOL 507 » par ISP,
Glyceryl PABA,
PEG-25 PABA vendu sous le nom « UVINUL P25 » par BASF,

### Dérivés salicyliques :

Homosalate vendu sous le nom « Eusolex HMS » par Rona/EM Industries,
Ethylhexyl Salicylate vendu sous le nom « NEO HELIOPAN OS » par SYMRISE,
Dipropyleneglycol Salicylate vendu sous le nom « DIPSAL » par SCHER,
TEA Salicylate, vendu sous le nom « NEO HELIOPAN TS » par SYMRISE,

### Dérivés de β,β-diphénylacrylate :

Octocrylene vendu notamment sous le nom commercial « UVINUL N539 » par BASF,
Etocrylene, vendu notamment sous le nom commercial « UVINUL N35 » par BASF,

### Dérivés de la benzophénone :

Benzophenone-1 vendu sous le nom commercial « UVINUL 400 » par BASF,
Benzophenone-2 vendu sous le nom commercial « UVINUL D50 » par BASF
Benzophenone-3 ou Oxybenzone, vendu sous le nom commercial « UVINUL M40 » par BASF,
Benzophenone-4 vendu sous le nom commercial « UVINUL MS40 » par BASF, Benzophenone-5
Benzophenone-6 vendu sous le nom commercial « Helisorb 11 » par Norquay
Benzophenone-8 vendu sous le nom commercial « Spectra-Sorb UV-24 » par American Cyanamid
Benzophenone-9 vendu sous le nom commercial« UVINUL DS-49» par BASF, Benzophenone-12
2-(4-diethylamino-2-hydroxybenzoyl)-benzoate de n-hexyle vendu sous le nom commercial « UVINUL A+ » par BASF.

### Dérivés du benzylidène camphre :

3-Benzylidene camphor fabriqué sous le nom « MEXORYL SD» par CHIMEX,
4-Methylbenzylidene camphor vendu sous le nom « EUSOLEX 6300 » par MERCK,
Benzylidene Camphor Sulfonic Acid fabriqué sous le nom « MEXORYL SL» par CHIMEX,
Camphor Benzalkonium Methosulfate fabriqué sous le nom « MEXORYL SO » par CHIMEX,
Terephthalylidene Dicamphor Sulfonic Acid fabriqué sous le nom « MEXORYL SX » par CHIMEX,
Polyacrylamidomethyl Benzylidene Camphor fabriqué sous le nom « MEXORYL SW » par CHIMEX,

### Dérivés du phenyl benzimidazole :

Phenylbenzimidazole Sulfonic Acid vendu notamment sous le nom commercial « EUSOLEX 232 » par MERCK,
Disodium Phenyl Dibenzimidazole Tetra-sulfonate vendu sous le nom commercial « NEO HELIOPAN AP » par SYMRISE,

### Dérivés du phenyl benzotriazole :

Drometrizole Trisiloxane vendu sous le nom « Silatrizole » par RHODIA CHIMIE Methylène bis-Benzotriazolyl Tetramethylbutylphénol, vendu sous forme solide sous le nom commercial « MIXXIM BB/100 » par FAIRMOUNT CHEMICAL ou sous forme micronisée en dispersion aqueuse sous le nom commercial « TINOSORB M » par CIBA SPECIALTY CHEMICALS,

### Dérivés de triazine :

- Bis-Ethylhexyloxyphenol Methoxyphenyl Triazine vendu sous le nom commercial «TINOSORB S » par CIBA GEIGY,
- Ethylhexyl triazone vendu notamment sous le nom commercial «UVINUL T150 » par BASF,
- Diethylhexyl Butamido Triazone vendu sous le nom commercial « UVASORB HEB » par SIGMA 3V,
- la 2,4,6-tris(4'-amino benzalmalonate de dinéopentyle)-s-triazine
- la 2,4,6-tris-(4'-amino benzalmalonate de diisobutyle)-s- triazine,
- la 2,4bis (4'-aminobenzalmalonate de dinéopentyle)-6-(4'-aminobenzoate de n-butyle)-s-triazine,
- les filtres triazines symétriques décrits dans le brevet US6,225,467, la demande W02004/085412 (voir composés 6 et 9) ou le document « Symetrical Triazine Derivatives » IP.COM Journal , IP.COM INC WEST HENRIETTA, NY, US (20 septembre 2004) notamment les 2,4,6-tris-(biphényl)-1,3,5-triazines (en particulier la 2,4,6-tris(biphenyl-4-yl-1,3,5-triazine et la 2,4,6-tris(terphenyl)-1,3,5-triazine qui est repris dans les demandes de brevet WO06/035000, WO06/034982, WO06/034991, WO06/035007, W02006/034992, WO2006/034985.

### Dérivés anthraniliques :

Menthyl anthranilate vendu sous le nom commercial commercial « NEO HELIOPAN MA » par SYMRISE,

### Dérivés d'imidazolines :

Ethylhexyl Dimethoxybenzylidene Dioxoimidazoline Propionate,

### Dérivés du benzalmalonate :

Polyorganosiloxane à fonctions benzalmalonate comme le Polysilicone-15 vendu sous la dénomination commerciale « PARSOL SLX » par DSM Nutritional Products, Inc

### Dérivés de 4,4-diarvlbutadiène :

-1,1-dicarboxy (2,2'-diméthyl-propyl)-4,4-diphénylbutadiène

### Dérivés de benzoxazole :

2,4-bis-[5-1(diméthylpropyl)benzoxazol-2-yl-(4-phenyl)-imino]-6-(2-ethylhexyl)-imino-1,3,5-triazine vendu sous le nom d'Uvasorb K2A par Sigma 3V et leurs mélanges.

### Dérivés de mérocyanine

Octyl-5-N,N-diethylamino-2-phenysulfonyl-2,4-pentadienoate

Les filtres organiques préférentiels sont choisis parmi
Ethylhexyl Methoxycinnamate
Ethylhexyl Salicylate,
Homosalate,
Octocrylene,
Phenylbenzimidazole Sulfonic Acid,
Benzophenone-3,
Benzophenone-4,
Benzophenone-5,
2-(4-diethylamino-2-hydroxybenzoyl)-benzoate de n-hexyle.
4-Methylbenzylidene camphor,
Terephthalylidene Dicamphor Sulfonic Acid,
Disodium Phenyl Dibenzimidazole Tetra-sulfonate,
Methylène bis-Benzotriazolyl Tetramethylbutylphénol
Bis-Ethylhexyloxyphenol Methoxyphenyl Triazine
Ethylhexyl triazone,
Diethylhexyl Butamido Triazone,
la 2,4,6-tris(4'-amino benzalmalonate de dinéopentyle)-s-triazine
la 2,4,6-tris-(4'-amino benzalmalonate de diisobutyle)-s- triazine
la 2,4bis (4'-aminobenzoate de n-butyle)-6-(aminopropyltrisiloxane)-s-triazine,
la 2,4bis (4'-aminobenzalmalonate de dinéopentyle)-6-(4'-aminobenzoate de n-butyle)-s-triazine,
la 2,4,6-tris(biphenyl-4-yl-1,3,5-triazine
la 2,4,6-tris(terphenyl)-1,3,5-triazine
Drometrizole Trisiloxane
Polysilicone-15
1,1-dicarboxy (2,2'-diméthyl-propyl)-4,4-diphénylbutadiène
2,4-bis-[5-1(diméthylpropyl)benzoxazol-2-yl-(4-phenyl)-imino]-6-(2-ethylhexyl)-imino-1,3,5-triazine
Octyl-5-N,N-diethylamino-2-phenysulfonyl-2,4-pentadienoate et leurs mélanges.

Les filtres organiques complémentaires conformes à l'invention représentent en général de 0,1 à 30 %, de préférence de 1 à 25 %, du poids total de la composition.

Bien entendu, l'homme de l'art veillera à choisir le ou les éventuels filtres complémentaires et/ou leurs quantités de manière telle que les propriétés avantageuses attachées intrinsèquement aux compositions conformes à l'invention ne soient pas, ou substantiellement pas, altérées par la ou les adjonctions envisagées.

Les compositions selon l'invention peuvent également contenir des agents de bronzage et/ou de brunissage artificiels de la peau (agents autobronzants), et plus particulièrement la dihydroxyacétone (DHA). Ils sont présents de préférence dans des quantité allant 0,1 à 10% en poids par rapport au poids total de la composition.

Les compositions aqueuses conformes à la présente invention peuvent comprendre en outre des adjuvants cosmétiques classiques notamment choisis parmi les corps gras, les solvants organiques, les épaississants ioniques ou non ioniques, hydrophiles ou lipophiles, les adoucissants, les humectants, les opacifiants, les stabilisants, les émollients, les silicones, les agents anti-mousse, les parfums, les conservateurs, les tensioactifs anioniques, cationiques, non-ioniques, zwitterioniques ou amphotères, des actifs, les charges, les polymères, les propulseurs, les agents alcalinisants ou acidifiants ou tout autre ingrédient habituellement utilisé dans le domaine cosmétique et/ou dermatologique.

Bien entendu, l'homme de l'art veillera à choisir le ou les éventuels additifs et/ou leurs quantités de manière telle que les propriétés avantageuses attachées intrinsèquement aux compositions conformes à l'invention ne soient pas, ou substantiellement pas, altérées par la ou les adjonctions envisagées.

Les corps gras peuvent être constitués par une huile ou une cire autre que les cires apolaires telles que définies précédemment ou leurs mélanges. Par huile, on entend un composé liquide à température ambiante. Par cire, on entend un composé solide ou substantiellement solide à température ambiante, et dont le point de fusion est généralement supérieur à 35°C.

Comme huiles, on peut citer les huiles minérales (paraffine); végétales (huile d'amande douce, de macadamia, de pépin de cassis, de jojoba) ; synthétiques comme le perhydrosqualène, les alcools, les amides grasses (comme l'isopropyl lauroyl sarcosinate vendu sous la dénomination d' « Eldew SL-205 » par la société Ajinomoto), les acides ou les esters gras comme le benzoate d'alcools en C12-C15 vendu sous la dénomination commerciale « Finsolv TN » ou « Witconol TN » par la société WITCO, le Benzoate de 2-éthylphenyle comme le produit commercial vendu sous le nom X-TEND 226 ® par la société ISP, le palmitate d'octyle, le lanolate d'isopropyle, les triglycérides dont ceux des acides caprique/caprylique, le dicaprylyl carbonate vendu sous la dénomination « Cetiol CC » par la société Cognis), les esters et éthers gras oxyéthylénés ou oxypropylénés; les huiles siliconées (cyclométhicone, polydiméthysiloxanes ou PDMS) ou fluorées, les polyalkylènes.

Comme composés cireux, on peut citer la cire de carnauba, la cire d'abeille, l'huile de ricin hydrogénée, les cires de polyéthylène et les cires de polyméthylène comme celle vendue sous la dénomination Cirebelle 303 par la société SASOL.

Parmi les solvants organiques, on peut citer les alcools et polyols inférieurs. Ces derniers peuvent être choisis parmi les glycols et les éthers de glycol comme l'éthylène glycol, le propylène glycol, le butylène glycol, le dipropylène glycol ou le diéthylène glycol.

Comme épaississants hydrophiles, on peut citer les polymères carboxyvinyliques tels que les Carbopols (Carbomers) et les Pemulen (Copolymère acrylate/C10-C30-alkylacrylate) ; les polyacrylamides comme par exemple les copolymères réticulés vendus sous les noms Sepigel 305 (nom C.T.F.A. : polyacrylamide/C13-14 isoparaffin/Laureth 7) ou Simulgel 600 (nom C.T.F.A. : acrylamide / sodium acryloyldimethyltaurate copolymer / isohexadecane / polysorbate 80) par la société Seppic ; les polymères et copolymères d'acide 2-acrylamido 2-méthylpropane sulfonique, éventuellement réticulés et/ou neutralisés, comme le poly(acide 2-acrylamido 2-méthylpropane sulfonique) commercialisé par la société Hoechst sous la dénomination commerciale « Hostacerin AMPS » (nom CTFA : ammonium polyacryloyldimethyl taurate ou le SIMULGEL 800 commercialisé par la société SEPPIC (nom CTFA : sodium polyacryolyldimethyl taurate / polysorbate 80 / sorbitan oleate) ; les copolymères d'acide 2-acrylamido 2-méthylpropane sulfonique et d'hydroxyethyl acrylate comme le SIMULGEL NS et le SEPINOV EMT 10 commercialisés par la société SEPPIC ; les dérivés cellulosiques tels que l'hydroxyéthylcellulose ; les polysaccharides et notamment les gommes telles que la gomme de Xanthane ; et leurs mélanges.

Comme épaississants lipophiles, on peut citer les polymères synthétiques tels que les poly C10-C30 alkyl acrylates vendu sous la dénomination « INTELIMER IPA 13-1 » et « INTELIMER IPA 13-6 » par la société Landec ou encore les argiles modifiées telles que l'hectorite et ses dérivés, comme les produits commercialisés sous les noms de Bentone.

Parmi les actifs, on peut citer :
- les vitamines (C, K, PP...) et leurs dérivés ou précurseurs, seuls ou en mélanges,
- les agents anti-pollution et/ou agent anti-radicalaire ;
- les agents dépigmentants et/ou des agents pro-pigmentants ;
- les agents anti-glycation ;
- les agents apaisants,
- les inhibiteurs de NO-synthase ;
- les agents stimulant la synthèse de macromolécules dermiques ou épidermiques et/ou empêchant leur dégradation ;
- les agents stimulant la prolifération des fibroblastes ;
- les agents stimulant la prolifération des kératinocytes ;
- les agents myorelaxants;
- les agents tenseurs,
- les agents matifiants,
- les agents kératolytiques,
- les agents desquamants ;
- les agents hydratants ;
- les agents anti-inflammatoires ;
- les agents agissant sur le métabolisme énergétique des cellules,
- les agents répulsifs contre les insectes
- les antagonistes de substances P ou de CRGP.
- les agents anti-chute et/ou repousse des cheveux
- les agents anti-rides.

Les compositions selon l'invention peuvent être préparées selon les techniques bien connues de l'homme de l'art. Elles peuvent se présenter en particulier sous forme d'émulsion, simple ou complexe (H/E, E/H, H/E/H ou E/H/E) telle qu'une crème, un lait ou d'un gel crème ; sous la forme d'un gel aqueux ; sous la forme d'une lotion. Elles peuvent éventuellement être conditionnées en aérosol et se présenter sous forme de mousse ou de spray.

De préférence, les compositions selon l'invention se présentent sous la forme d'une émulsion huile-dans-eau ou eau-dans huile.

Les émulsions contiennent généralement au moins un émulsionnant choisi parmi les émulsionnants amphotères, anioniques, cationiques ou non ioniques, utilisés seuls ou en mélange. Les émulsionnants sont choisis de manière appropriée suivant l'émulsion à obtenir (E/H ou H/E). Les émulsions peuvent contenir également d'autres types de stabilisants comme par exemple des charges, des polymères gélifiants ou épaississants.

Comme tensioactifs émulsionnants utilisables pour la préparation des émulsions E/H, on peut citer par exemple les alkyl esters ou éthers de sorbitane, de glycérol ou de sucres ; les tensioactifs siliconés comme les diméthicone copolyols tels que le mélange de cyclométhicone et de diméthicone copolyol, vendu sous la dénomination « DC 5225 C » par la société Dow Corning, et les alkyl-dimethicone copolyols tels que le Laurylmethicone copolyol vendu sous la dénomination "Dow Corning 5200 Formulation Aid" par la société Dow Corning ; le Cetyl dimethicone copolyol tel que le produit vendu sous la dénomination Abil EM 90R par la société Goldschmidt et le mélange de cétyl diméthicone copolyol, d'isostéarate de polyglycérole (4 moles) et de laurate d'hexyle vendu sous la dénomination ABIL WE 09 par la société Goldschmidt. On peut y ajouter aussi un ou plusieurs co-émulsionnants, qui, de manière avantageuse, peuvent être choisis dans le groupe comprenant les esters alkylés de polyol.

Comme esters alkylés de polyol, on peut citer notamment les esters de polyéthylèneglycol comme le PEG-30 Dipolyhydroxystearate tel que le produit commercialisé sous le nom Arlacel P135 par la socité ICI .

Comme esters de glycérol et/ou de sorbitan, on peut citer par exemple l'isostéarate de polyglycérol, tel que le produit commercialisé sous la dénomination Isolan GI 34 par la société Goldschmidt ; l'isostéarate de sorbitan, tel que le produit commercialisé sous la dénomination Arlacel 987 par la société ICI ; l'isostéarate de sorbitan et le glycérol, tel que le produit commercialisé sous la dénomination Arlacel 986 par la société ICI, et leurs mélanges.

Pour les émulsions H/E, on peut citer par exemple comme émulsionnants, les émulsionnants non ioniques tels que les esters d'acides gras et de glycérol oxyalkylénés (plus particulièrement polyoxyéthylénés) ; les esters d'acides gras et de sorbitan oxyalkylénés ; les esters d'acides gras oxyalkylénés (oxyéthylénés et/ou oxypropylénés) comme le mélange PEG-100 Stearate/ Glyceryl Stearate commercialisé par exemple par la société ICI sous la dénomination Arlacel 165 ; les éthers d'alcools gras oxyalkylénés (oxyéthylénés et/ou oxypropylénés) ; les esters de sucres comme le stéarate de sucrose ; les éthers d'alcool gras et de sucre, notamment les alkylpolyglucosides (APG) tels que le décylglucoside et le laurylglucoside commercialisés par exemple par la société Henkel sous les dénominations respectives Plantaren 2000 et Plantaren 1200, le cétostéarylglucoside éventuellement en mélange avec l'alcool cétostéarylique, commercialisé par exemple sous la dénomination Montanov 68 par la société Seppic, sous la dénomination Tegocare CG90 par la société Goldschmidt et sous la dénomination Emulgade KE3302 par la société Henkel, ainsi que l'arachidyl glucoside, par exemple sous la forme du mélange d'alcools arachidique et béhénique et d'arachidylglucoside commercialisé sous la dénomination Montanov 202 par la société Seppic. Selon un mode particulier de réalisation de l'invention, le mélange de l'alkylpolyglucoside tel que défini ci-dessus avec l'alcool gras correspondant peut être sous forme d'une composition auto-émulsionnante, comme décrit par exemple dans le document WO-A-92/06778.

Parmi les autres stabilisants d'émulsion, on utilisera plus particulièrement les polymères d'acide isophtalique ou d'acide sulfoisophtalique, et en particulier les copolymères de phtalate / sulfoisophtalate / glycol par exemple le copolymère de Diéthylèneglycol / Phtalate / Isophtalate / 1,4-cyclohexane-diméthanol (nom INCI : Polyester-5) vendu sous les dénominations "Eastman AQ polymer" (AQ35S, AQ38S, AQ55S, AQ48 Ultra) par la société Eastman Chemical.

Lorsqu'il s'agit d'une émulsion, la phase aqueuse de celle-ci peut comprendre une dispersion vésiculaire non ionique préparée selon des procédés connus (Bangham, Standish and Watkins. J. Mol. Biol. 13, 238 (1965), FR 2 315 991 et FR 2 416 008).

Les compositions selon l'invention trouvent leur application dans un grand nombre de traitements, notamment cosmétiques, de la peau, des lèvres et des cheveux, y compris le cuir chevelu, notamment pour la protection et/ou le soin de la peau, des lèvres et/ou des cheveux, et/ou pour le maquillage de la peau et/ou des lèvres.

Un autre objet de la présente invention est constitué par l'utilisation des compositions selon l'invention telles que ci-dessus définies pour la fabrication de produits pour le traitement cosmétique de la peau, des lèvres, des ongles, des cheveux, des cils, sourcils et/ou du cuir chevelu, notamment des produits de soin, des produits de protection solaire et des produits de maquillage.

Les compositions cosmétiques selon l'invention peuvent par exemple être utilisées comme produit de maquillage.

Les compositions cosmétiques selon l'invention peuvent par exemple être utilisées comme produit de soin et/ou de protection solaire pour le visage et/ou le corps de consistance liquide à semi-liquide, telles que des laits, des crèmes plus ou moins onctueuses, gel-crèmes, des pâtes. Elles peuvent éventuellement être conditionnées en aérosol et se présenter sous forme de mousse ou de spray.

Les compositions selon l'invention sous forme de lotions fluides vaporisables conformes à l'invention sont appliquées sur la peau ou les cheveux sous forme de fines particules au moyen de dispositifs de pressurisation. Les dispositifs conformes à l'invention sont bien connus de l'homme de l'art et comprennent les pompes non-aérosols ou "atomiseurs", les récipients aérosols comprenant un propulseur ainsi que les pompes aérosols utilisant l'air comprimé comme propulseur. Ces derniers sont décrits dans les brevets US 4,077,441 et US 4,850,517 (faisant partie intégrante du contenu de la description).

Les compositions conditionnées en aérosol conformes à l'invention contiennent en général des agents propulseurs conventionnels tels que par exemple les composés hydrofluorés le dichlorodifluorométhane, le difluoroéthane, le diméthyléther, l'isobutane, le n-butane, le propane, le trichlorofluorométhane. Ils sont présents de préférence dans des quantités allant de 15 à 50% en poids par rapport au poids total de la composition.

Les exemples qui suivent servent à illustrer l'invention. Dans ces exemples, les quantités des ingrédients compositions sont données en % pondéral par rapport au poids total de la composition.

### EXEMPLES 1 à 4

### 1. Photostabilité du dibenzoylméthane

On a évalué l'effet photostabilisant des composés esters de 2-pyrrolidinone 4-carboxy de formule (I) conformes à l'invention vis-à-vis du dérivé de dibenzoylméthane : Butyl Methoxy Dibenzoylmethane (avobenzone), proposé à la vente sous la dénomination commerciale de "PARSOL 1789" par la Société Roche Vitamins (exemples 1, 2, 3 et 4).

On a comparé également l'effet photostabilisant de ces dérivés esters de 2-pyrrolidinone 4-carboxy avec celui de l'Isopropyl Lauroyl Sarcosinate (exemple A) de formule :

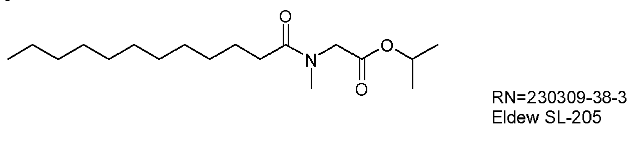

L'exemple B ne contient ni de composé ester de pyrrolidone-4-carboxy ni l'isopropyl lauroyl sarcosinate.

| **Phase** | **Ingrédients** | **A** | **B** | **1** | **2** | **3** | **4** |
|---|---|---|---|---|---|---|---|
| Grasse | C₁₂-C₁₅ alkyl benzoate | 10,0 | 10,0 | 10,0 | 10,0 | 10,0 | 10,0 |
| | Alcool cétéarylique et cétéaryl glucoside | 7,5 | 7,5 | 7,5 | 7,5 | 7,5 | 7,5 |
| | Butyl Methoxy Dibenzoylmethane | 1,0 | 1,0 | 1,0 | 1,0 | 1,0 | 1,0 |
| | Isopropyl Lauroyl Sarcosinate | 10,0 | - | - | - | - | - |
| | Composé j | - | - | 10,0 | - | - | - |
| | Composé I | - | - | - | 10,0 | - | - |
| | Composé m | - | - | - | - | 10,0 | - |
| | Composé n | 5,0 | 5,0 | 5,0 | 5,0 | 5,0 | 10,0 |
| Aqueuse | Eau | qsp 100 | qsp 100 | qsp 100 | qsp 100 | qsp 100 | qsp 100 |

### PRINCIPE DE LA METHODE :

On mesure le pourcentage en perte de dérivé de dibenzoylméthane induite par l'exposition à un simulateur solaire d'une formule étalée en films d'épaisseur voisine de 20µm. L'évaluation est faite par analyse HPLC du filtre en solution, après extraction des films, en comparant des échantillons irradiés et non irradiés.

### MATERIEL ET METHODE :

Simulateur solaire : appareil Oriel 1000W muni d'une sortie 4 pouces, avec filtre 81017 et miroir dichroïque. Les échantillons sont exposés en position horizontale. L'UV-mètre utilisé est l'appareil OSRAM CENTRA équipé de 2 têtes de lecture, l'une pour l'UVA l'autre pour l'UVB. L'ensemble simulateur - UV mètre est étalonné annuellement par spectroradiométrie. Les mesures d'irradiance sont effectuées en début et en fin d'exposition en plaçant les têtes de lecture à la position de l'échantillon. Les irradiances sont :
0,35 - 0,45 mW/cm² en UVB
16 - 18 mW/cm² en UVA

Le Butyl Methoxy Dibenzoylmethane résiduel est mesuré par Chromatographie : chaîne HPLC avec détecteur à barette de diodes. Les dérivés esters de 2-pyrrolidinone 4-carboxy sont introduits dans le support commun défini ci-dessus à une concentration de 10% en présence de 1 % de Butyl Methoxydibenzoylméthane de composition : On mesure également la perte en Butyl Methoxy Dibenzoylméthane (avobenzone) après irradiation dans le même support ne contenant pas de composé de formule (I) (Composition B)

### MISE EN OEUVRE DES TESTS DE PHOTOSTABILITE

Environ 20mg de chaque formule testée sont étalés sur une surface de 10 cm² sur une face dépolie d'un disque de silice fondue. La quantité exacte est déterminée par pesée. 3 films sont exposés au simulateur solaire et 3 autres servent de témoin. Les échantillons sont exposés 3 par 3 à la lumière du simulateur solaire pendant un temps suffisant pour délivrer une dose UVA égale à 12J corrigés de la sensibilité de l'UV mètre correspondant à la dégradation de environ 50% du Butyl Methoxy Dibenzoylméthane (avobenzone) en l'absence d'agent photoprotecteur. A la fin de l'exposition chaque disque support est introduit dans un bocal de 600ml avec 10ml d'un solvant approprié (en général EtOH) ; l'ensemble est placé pendant 5 minutes dans une cuve à ultrasons. La solution est ensuite transférée dans des flacons adaptés au support compatible avec l'appareil d'analyse HPLC utilisé. Les conditions analytiques peuvent être ajustées en fonction de l'actif testé. Le calcul des taux résiduels est effectué à partir des moyennes obtenues sur les échantillons irradiés et non irradiés, comme décrit ci-dessous :

**TABLEAU I**

| **Exemples** | **Composé amidé** | **% taux résiduel en avobenzone** |
|---|---|---|
| **Exemple A** | ELDEW SL-205 | 64.2 ± 0.45 |
| **Exemple B** | aucun | 19.8 ± 0.8 |
| **Exemple 1** | Composé (j) | 72.8 ± 2.0 |
| **Exemple 2** | Composé (l) | 74.5 ± 2.7 |
| **Exemple 3** | Composé (m) | 78.5 ± 1.4 |
| **Exemple 4** | Composé (n) | 70.9 ± 1.3 |

Les composés esters de 2-pyrrolidinone 4-carvboxy de formules (j), (1), (m), (n), des exemples 1 à 6 selon l'invention ont un effet photostabilisant vis-à-vis du dérivé de dibenzoylméthane supérieur à celui obtenu avec l'Isopropyl Lauroyl Sarcosinate.

### 2. Stabilité galénique des compositions:

On compare les stabilités thermiques des exemples 1, 2, 3 et 4 correspondant aux dérivés de l'invention avec la formule C identique à la formule A mais contenant à la place de l'Isopropyl Lauroyl Sarcosinate le composé amidé N-butyl N-acétyl aminopropionate d'éthyle (R3535 - MERCK). On observe à l'oeil nu (aspect macroscopique) l'aspect de chaque formule après 2 mois de stockage dans une étude à 45°C.

| **Compositions** | **Aspect macroscopique et microscopique au temps 2 mois à 45°C** |
|---|---|
| Formule 1 comprenant le composé (j) | L'émulsion reste stable et homogène |
| Formule 2 comprenant le composé (I) | L'émulsion reste stable et homogène |
| Formule 3 comprenant le composé (m) | L'émulsion reste stable et homogène |
| Formule 4 comprenant le composé (n) | L'émulsion reste stable et homogène |
| Formule C comprenant le N-butyl N-acétyl aminopropionate d'éthyle (R3535) | On observe un déphasage |

On observe clairement que, comparé au N-butyl N-acétyl aminopropionate d'éthyle (R3535) tel que décrit dans l'EP717982, les composés (j), (l), (m et (n) de formule (I) selon l'invention permettent d'obtenir un bon état de dispersion de l'huile dans la composition et au final une bonne stabilité galénique.

## Revendications

1. Composition comprenant dans un support cosmétiquement acceptable au moins un système filtrant UV, **caractérisée par le fait qu'**elle comprend :
(a) au moins un dérivé du dibenzoylméthane et
(b) au moins un composé ester de 2-pyrrolidinone 4-carboxy de formule (I) suivante : 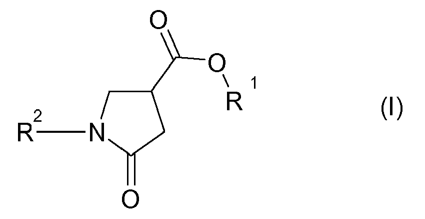
dans laquelle :
R¹ désigne un radical alkyle en C₁-C₂₀, linéaire ou ramifié.
R² désigne un radical alkyle en C₁-C₂₀, linéaire ou ramifié pouvant contenir un cycle en C₅-C₆, le radical phényle, le radical benzyle ou le radical phénéthyle.

2. Composition selon la revendication 1, où le composé de formule (I), est choisi parmi les composés (a) à (oo) suivants :

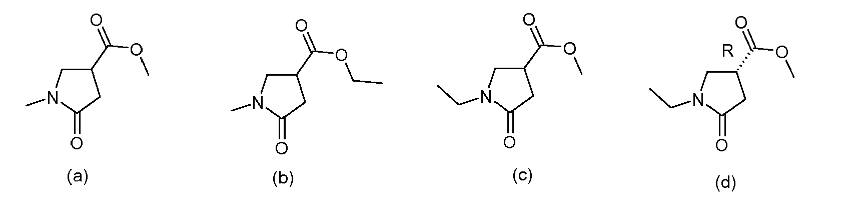

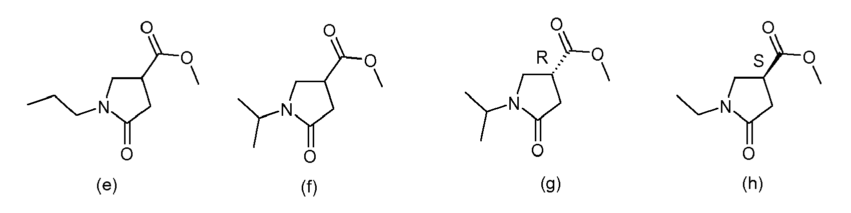

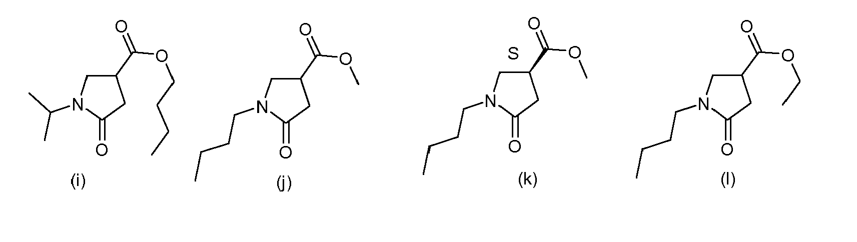

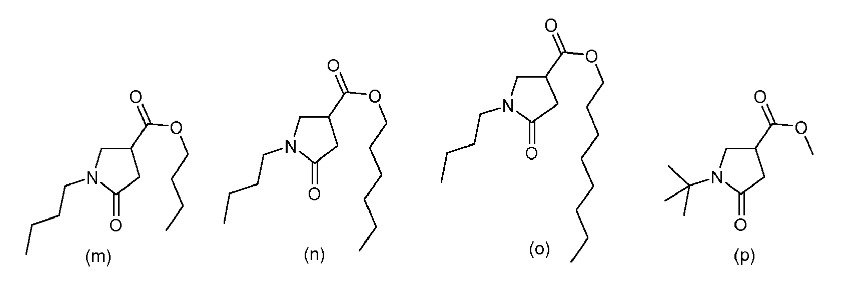

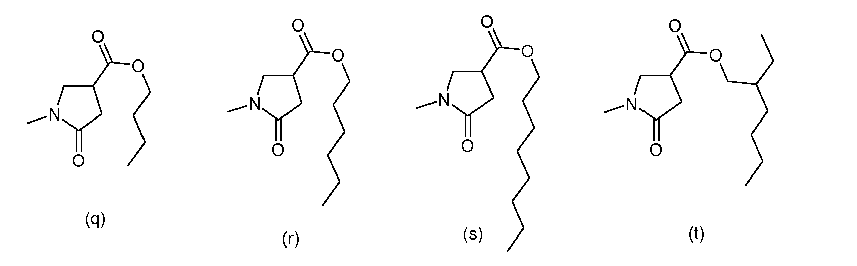

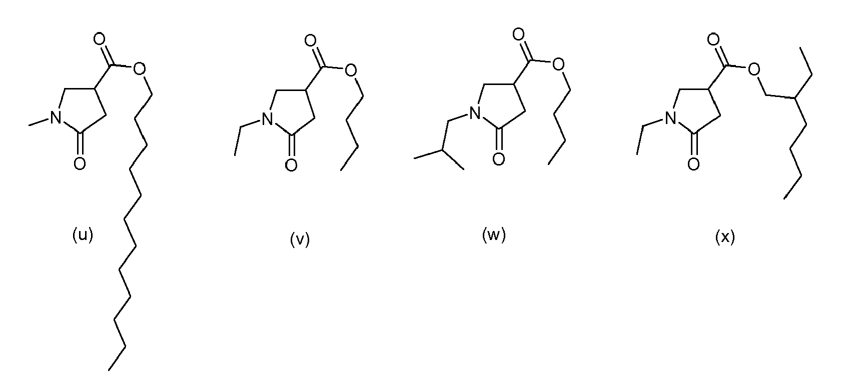

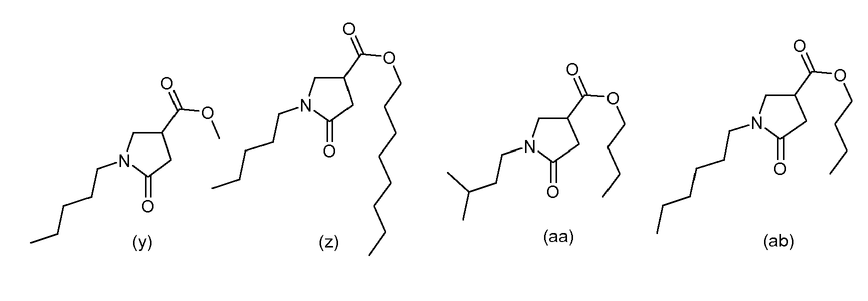

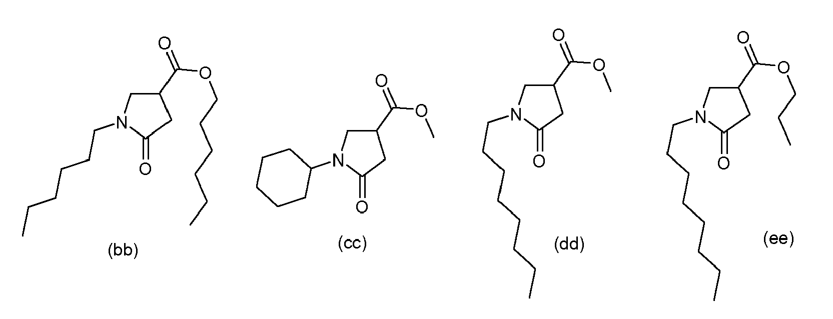

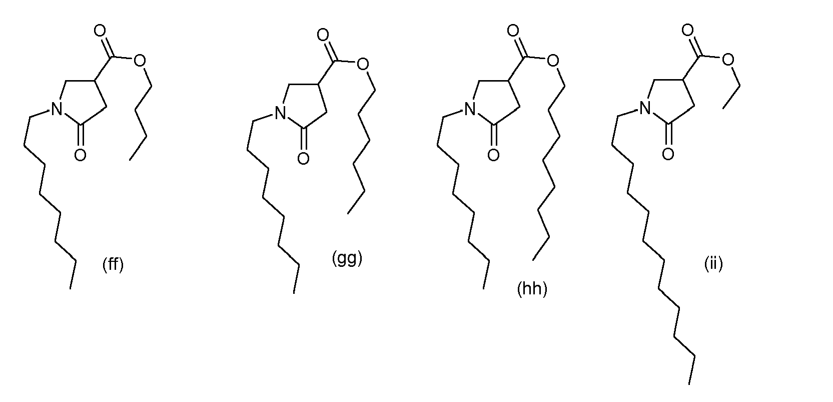

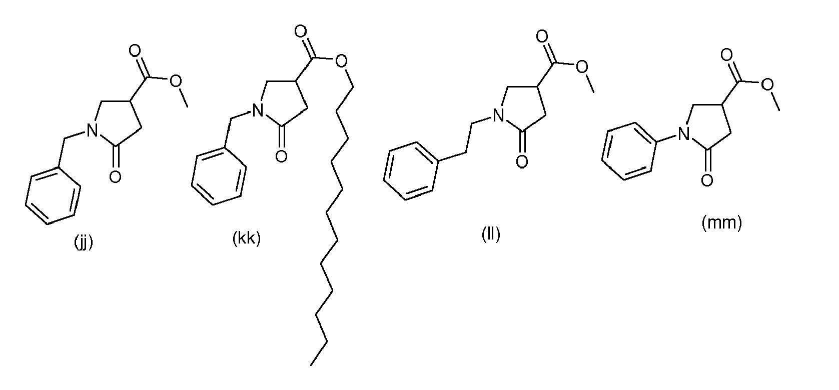

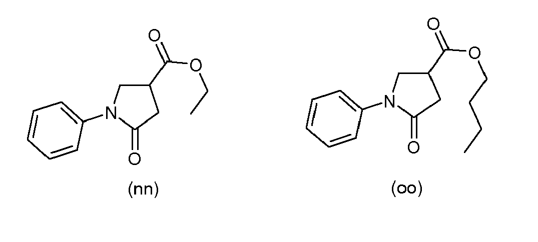

3. Composition selon la revendication 2, où le composé de formule (I), est choisi parmi les composés

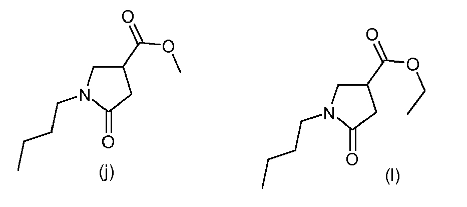

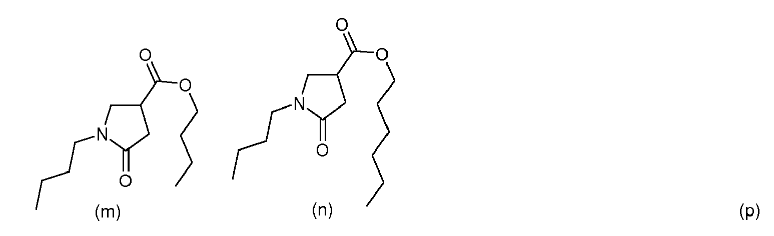

4. Composition selon l'une quelconque des revendications 1 à 3, où le dérivé de dibenzoylméthane est le 4-(ter.-butyl) 4'-méthoxy dibenzoylméthane ou Butyl Methoxy Dibenzoylmethane de formule suivante :

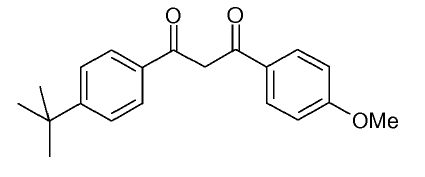

5. Composition selon l'une quelconque des revendications 1 à 3, **caractérisée par le fait qu'**elle contient en plus un composé s-triazine silicié et substitué par deux groupements aminobenzoates ou aminobenzamides de formule générale (VI) suivante ou l'une de ses formes tautomères : 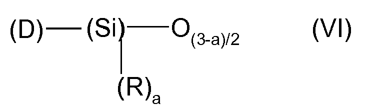 dans laquelle
- R, identiques ou différents représentent un radical alkyle en C₁-C₃₀, linéaire ou ramifié et éventuellement halogéné ou insaturé, un radical aryle en C₆-C₁₂, un radical alkoxy en C₁-C₁₀, un radical hydroxy ou le groupe triméthylsilyloxy ;
- a = 0 à 3 ; en plus des unités de formule -A-(Si)(R)ₐ(O)_{(3-a)/2}, l'organosiloxane peut comporter des unités de formule : (R)_{b}-(Si)(O)_{(4-b)/2} dans lesquelles : R a la même signification que dans la formule (II), b = 1, 2 ou 3 ;
- le groupe (D) désigne un composé s-triazine de formule (VII) suivante : 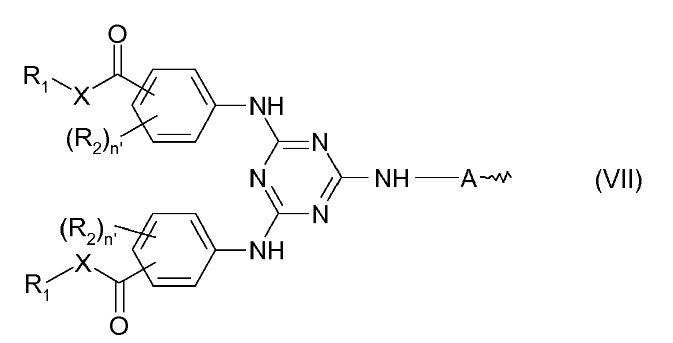
où
- X représente -O- ou -NR₃-, avec R₃ qui représente l'hydrogène ou un radical alkyle en C₁-C₅,
- R₁ représente un radical alkyle en C₁-C₃₀, linéaire ou ramifié et éventuellement insaturé et pouvant contenir un atome de silicium, un groupe cycloalkyle en C₅-C₂₀, éventuellement substitué par 1 à 3 radicaux alkyles en C₁-C₄, linéaires ou ramifiés, le groupe -(CH₂CHR₄-O)ₘR₅ ou le groupe -CH₂-CH(OH)-CH₂-O-R₆,
- R₄ représente l'hydrogène ou méthyle ; le groupement (C=O)XR, pouvant être en position ortho, méta ou para du groupement amino,
- R₅ représente l'hydrogène ou un groupe alkyle en C₁-C₈,
- R₆ représentent l'hydrogène ou un groupe alkyle en C₄-C₈,
- m est un nombre entier allant de 2 à 20,
- n' = 0 à 2,
- R₂, identiques ou différents, représentent un radical hydroxy, un radical alkyle en C₁-C₈, linéaire ou ramifié, un radical alcoxy en C₁-C₈, deux R₂ adjacents d'un même noyau aromatique pouvant former ensemble un groupement alkylidène dioxy dans lequel le groupe alkylidène contient 1 ou 2 atomes de carbone,
- A est un radical divalent choisi parmi méthylène, -[CH(Si(CH₃)₃]-, éthylène ou un groupe répondant à l'une des formules (VIII), (IX), (X) ou (XI) suivantes : 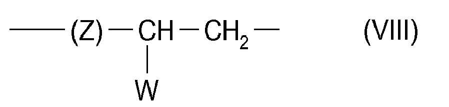 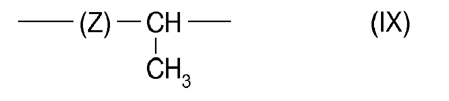
-(Z)-CH=CH- (X) 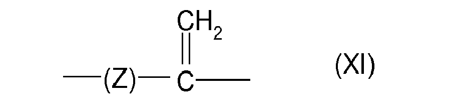
dans lesquelles :
- Z est un diradical alkylène en C₁-C₁₀, linéaire ou ramifié, saturé ou insaturé, éventuellement substitué par un radical hydroxyle ou des oxygènes et pouvant éventuellement contenir un groupement amino,
- W représente un atome d'hydrogène, un radical hydroxyle ou un radical alkyle en C₁-C₈, linéaire ou ramifié, saturé ou insaturé.

6. Composition selon la revendication 5 où le composé de formule (VI) est le 2,4-bis(4'-aminobenzoate de n-butyle)-6-[(3-{1,3,3,3-tetramethyl-1-[(trimethylsilyl)oxy]disiloxanyl}propyl)amino]-s-triazine de formule (5) :

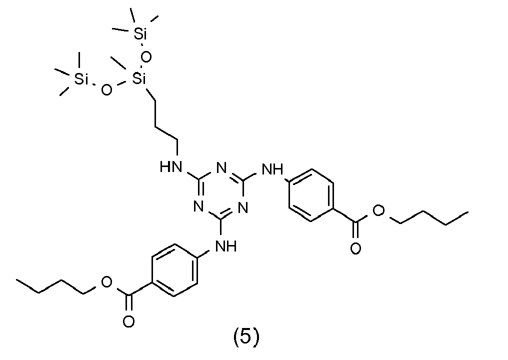

7. Composition selon l'une quelconque des revendications 1 à 6, **caractérisée par le fait qu'**elle contient en plus d'autres filtres organiques ou inorganiques actifs dans l'UV-A et/ou l'UV-B hydrosolubles ou liposolubles ou bien insolubles dans les solvants cosmétiques couramment utilisés.

8. Composition selon la revendication 7, où les filtres organiques complémentaires sont choisis parmi les anthranilates ; les dérivés cinnamiques ; les dérivés salicyliques, les dérivés du camphre ; les dérivés de la benzophénone ; les dérivés de P,P-diphénylacrylate , les dérivés de triazine ; les dérivés de benzotriazole ; les dérivés de benzalmalonate ; les dérivés de benzimidazole ; les imidazolines ; les dérivés bis-benzoazolyle ; les dérivés de l'acide p-aminobenzoïque (PABA) ; les dérivés de méthylène bis-(hydroxyphényl benzotriazole) ; les dérivés de benzoxazole ; les polymères filtres et silicones filtres ; les dimères dérivés d'a-alkylstyrène ; les 4,4-diarylbutadiènes , les dérivés de mérocyanines et leurs mélanges.

9. Composition selon la revendication 8, **caractérisée par le fait que** le ou les filtres UV organiques sont choisis parmi les composés suivants :
Ethylhexyl Methoxycinnamate
Ethylhexyl Salicylate,
Homosalate,
Octocrylene,
Phenylbenzimidazole Sulfonic Acid,
Benzophenone-3,
Benzophenone-4,
Benzophenone-5,
2-(4-diethylamino-2-hydroxybenzoyl)-benzoate de n-hexyle.
4-Methylbenzylidene camphor,
Terephthalylidene Dicamphor Sulfonic Acid,
Disodium Phenyl Dibenzimidazole Tetra-sulfonate,
Methylène bis-Benzotriazolyl Tetramethylbutylphénol
Bis-Ethylhexyloxyphenol Methoxyphenyl Triazine
Ethylhexyl triazone,
Diethylhexyl Butamido Triazone,
la 2,4,6-tris(4'-amino benzalmalonate de dinéopentyle)-s-triazine
la 2,4,6-tris-(4'-amino benzalmalonate de diisobutyle)-s- triazine
la 2,4bis (4'-aminobenzalmalonate de dinéopentyle)-6-(4'-aminobenzoate de n-butyle)-s-triazine,
la 2,4,6-tris(biphenyl-4-yl-1,3,5-triazine
la 2,4,6-tris(terphenyl)-1,3,5-triazine
Drometrizole Trisiloxane
Polysilicone-15
1,1-dicarboxy (2,2'-diméthyl-propyl)-4,4-diphénylbutadiène
2,4-bis-[5-1(diméthylpropyl)benzoxazol-2-yl-(4-phenyl)-imino]-6-(2-ethylhexyl)-imino-1,3,5-triazine
Octyl-5-N,N-diethylamino-2-phenysulfonyl-2,4-pentadienoate et leurs mélanges.

10. Composition selon la revendication 7, **caractérisée par le fait que** les filtres inorganiques complémentaires sont des pigments d'oxydes métalliques, enrobés ou non.

11. Composition selon la revendication 10, où les pigments d'oxydes métalliques enrobés ou non ont une taille moyenne de particule primaire: comprise entre 5 nm et 100 nm et de préférence entre 10 nm et 50 nm.

12. Composition selon la revendication 10 ou 11, où les pigments d'oxydes métalliques enrobés ou non sont choisis parmi des pigments d'oxyde de titane (amorphe ou cristallisé sous forme rutile et/ou anatase), de fer, de zinc, de zirconium ou de cérium ou leurs mélanges.

13. Composition selon l'une quelconque des revendications 1 à 12, **caractérisée par le fait qu'**elle se présente sous la forme d'une émulsion huile-dans-eau ou eau-dans huile.

14. Procédé pour améliorer la stabilité chimique vis-à-vis du rayonnement UV d'au moins un dérivé du dibenzoylméthane tel que défini dans l'une quelconque des revendications précédentes, **caractérisé par le fait qu'**on associe audit dérivé de dibenzoylméthane une quantité efficace d'au moins un composé ester de 2-pyrrolidinone 4-carboxy de formule (I) de formule (I) tel que défini dans les revendications précédentes.

15. Utilisation d'au moins un composé ester de 2-pyrrolidinone 4-carboxy de formule (I) tel que défini dans les revendications précédentes dans une composition comprenant dans un support cosmétiquement acceptable, au moins un dérivé du dibenzoylméthane tel que défini dans l'une quelconque des revendications précédentes dans le but de d'améliorer l'efficacité de ladite composition vis-à-vis des rayons UV-A.
